# EUROPEAN PATENT SPECIFICATION

(11) **EP 3 846 824 B1**
(45) Date of publication and mention of the grant of the patent: **18.06.2025**
(21) Application number: 19865804.9
(22) Date of filing: 06.09.2019
(51) Int. Cl.: A61K 47/69, A61K 31/7088, A61K 31/721, A61K 33/26

(54) **COMPOSITIONS AND METHODS FOR IMMUNE CHECKPOINT INHIBITION**
ZUSAMMENSETZUNGEN UND VERFAHREN ZUR IMMUNCHECKPOINT-INHIBITION
COMPOSITIONS ET MÉTHODES D'INHIBITION DE POINTS DE CONTRÔLE IMMUNITAIRES

(30) Priority: 07.09.2018 US 201862728459 P; 10.01.2019 US 201962790953 P
(43) Date of publication of application: 14.07.2021
(73) Proprietor: The General Hospital Corporation, Boston, Massachusetts 02114 (US)
(72) Inventor: MEDAROVA, Zdravka, Methuen, Massachusetts 01844 (US); YOO, Byunghee, Lexington, Massachusetts 02420-2341 (US)
(74) Representative: Fish & Richardson P.C.
(86) International application number: PCT/US2019/050003
(87) International publication number: WO 2020/068398

(56) References cited:
- EP-A1- 2 961 386
- WO-A1-2018/071911
- WO-A1-2018/071911
- US-A1- 2018 055 781
- US-B2- 9 629 812
- LUO XIN ET AL: "Folic acid-functionalized polyethylenimine superparamagnetic iron oxide nanoparticles as theranostic agents for magnetic resonance imaging and PD-L1 siRNA delivery for gastric cancer", INTERNATIONAL JOURNAL OF NANOMEDICINE, vol. Volume 12, 26 July 2017 (2017-07-26), pages 5331 - 5343, XP055845193, Retrieved from the Internet <URL:https://www.dovepress.com/getfile.php?fileID=37638> DOI: 10.2147/IJN.S137245
- ZDRAVKA MEDAROVA ET AL: "In vivo imaging of siRNA delivery and silencing in tumors", NATURE MEDICINE, vol. 13, no. 3, 1 March 2007 (2007-03-01), pages 372 - 377, XP055031597, ISSN: 1078-8956, DOI: 10.1038/nm1486
- YOO BYUNGHEE ET AL: "Guidelines for Rational Cancer Therapeutics", FRONTIERS IN ONCOLOGY, vol. 7, 12 December 2017 (2017-12-12), XP055845608, Retrieved from the Internet <URL:https://www.frontiersin.org/articles/10.3389/fonc.2017.00310/> DOI: 10.3389/fonc.2017.00310
- YOO BYUNGHEE ET AL: "Combining miR-10b-Targeted Nanotherapy with Low-Dose Doxorubicin Elicits Durable Regressions of Metastatic Breast Cancer", CANCER RESEARCH, vol. 75, no. 20, 10 September 2015 (2015-09-10), pages 4407 - 4415, XP055845609, ISSN: 0008-5472, Retrieved from the Internet <URL:https://cancerres.aacrjournals.org/content/75/20/4407.full-text.pdf> DOI: 10.1158/0008-5472.CAN-15-0888
- M. KUMAR ET AL: "Image-Guided Breast Tumor Therapy Using a Small Interfering RNA Nanodrug", CANCER RESEARCH, vol. 70, no. 19, 11 August 2010 (2010-08-11), pages 7553 - 7561, XP055229697, ISSN: 0008-5472, DOI: 10.1158/0008-5472.CAN-10-2070
- GHOSH SUBRATA K. ET AL: "Sequence-dependent combination therapy with doxorubicin and a survivin-specific small interfering RNA nanodrug demonstrates efficacy in models of adenocarcinoma : Sequence-dependent combination therapy for breast cancer", INTERNATIONAL JOURNAL OF CANCER, vol. 134, no. 7, 15 October 2013 (2013-10-15), US, pages 1758 - 1766, XP055845610, ISSN: 0020-7136, Retrieved from the Internet <URL:https://onlinelibrary.wiley.com/doi/epdf/10.1002/ijc.28499> DOI: 10.1002/ijc.28499
- YIGIT M V ET AL: "Context-dependent differences in miR-10b breast oncogenesis can be targeted for the prevention and arrest of lymph node metastasis", ONCOGENE, vol. 32, no. 12, 21 March 2013 (2013-03-21), London, pages 1530 - 1538, XP055845611, ISSN: 0950-9232, Retrieved from the Internet <URL:https://www.nature.com/articles/onc2012173.pdf> DOI: 10.1038/onc.2012.173
- JOSEPH S DOLINA ET AL: "Lipidoid Nanoparticles Containing PD-L1 siRNA Delivered In Vivo Enter Kupffer Cells and Enhance NK and CD8+ T Cell-mediated Hepatic Antiviral Immunity", MOLECULAR THERAPY-NUCLEIC ACIDS, vol. 2, 1 January 2013 (2013-01-01), US, pages e72, XP055727914, ISSN: 2162-2531, DOI: 10.1038/mtna.2012.63
- DOLINA ET AL.: "Lipidoid Nanoparticles Containing PD-L1 siRNA Delivered In Vivo Enter Kupffer Cells and Enhance NK and CD 8+ T Cell -mediated Hepatic Antiviral Immunity", MOLECULAR THERAPY-NUCLEIC ACIDS., vol. 2, 19 February 2013 (2013-02-19), pages e72, XP055727914, DOI: 10.1038/mtna.2012.63

## Description

### CLAIM OF PRIORITY

This application claims the benefit of U.S. Patent Application Serial No. 62/728,459, filed September 7, 2018, and 62/790,953, filed on January 10, 2019.

### TECHNICAL FIELD

Described herein are therapeutic compositions and the use of nanoparticles linked to inhibitory nucleic acids, e.g., siRNAs, targeting an immune checkpoint molecule, programmed cell death 1 ligand 1 (PD-L1) for treating cancer, e.g. pancreatic cancer.

### BACKGROUND

The recent past has seen impressive progress in the treatment of various malignancies using immunotherapy. One of the most promising approaches involves immune checkpoint inhibitors.

Despite the promise of checkpoint inhibition for cancer immunotherapy, the response is generally variable, with a large number of patients failing to respond. Notable examples of FDA approved PD-L1 inhibitors include atezolizumab for metastatic non-small cell lung cancer (NSCLC) ²¹, and durvalumab for locally advanced or metastatic urothelial carcinoma ²². However, despite initial encouraging results and fast-track approval of atezolizumab for bladder cancer ^{21,24}, the confirmatory trial failed to achieve its primary endpoint of overall survival ²⁵. Similarly, a phase III trial of durvalumab with tremelimumab as a first-line treatment of non-small cell lung cancer failed to meet its primary endpoint of progression-free survival ²⁶.

Luo, Xin, et al, International journal of nanomedicine (2017): 5331-5343, describes folic acid-functionalized polyethylenimine superparamagnetic iron oxide nanoparticles as theranostic agents for magnetic resonance imaging and PD-L1 siRNA delivery for gastric cancer.

### SUMMARY

The present invention is defined by the independent claims. The dependent claims depict other embodiments of the invention.

Any references in the description to methods of treatment, diagnosis or surgery refer to the compounds, pharmaceutical compositions and medicaments of the present invention for use in a method for treatment, diagnosis or surgery of the human or animal body. Any subject-matter falling outside the scope of the claims is provided for information purposes only.

Described herein are strategies for treating cancer that include combining chemotherapeutics such as gemcitabine and an immune checkpoint molecule inhibitor, a programmed death-ligand 1 (PD-L1) inhibitor, termed MN-siPDL1. As an example, MN-siPDL1 incorporates small interfering RNA against PD-L1 (siPDL1) conjugated to a magnetic nanocarrier (MN). As shown herein, delivery of MN-siPDL1to tumors can be monitored semi-quantitatively by noninvasive magnetic resonance imaging (MRI), because the MN carrier is superparamagnetic. Combination therapy consisting of chemotherapeutics such as gemcitabine and MN-siPDL1 in a syngeneic murine pancreatic cancer model resulted in a significant reduction in tumor growth and an increase in survival. Following dose optimization, a 90% reduction in tumor volume was achieved 3 weeks after the beginning of treatment. Whereas 100% of the control animals had succumbed to their tumors by week 6 after the beginning of treatment, there was no mortality in the experimental group by week 5, and 67% of the experimental animals survived for 12 weeks. These methods can be used to therapeutic benefit is an intractable disease for which there are no effective treatments and which is characterized by a mere 1% 5-year survival.

In accordance with the invention, provided herein are therapeutic nanoparticles, wherein said nanoparticles have a diameter of between 10 nm to 30 nm; and comprise an iron oxide core, a polymer coating comprising dextran, and an inhibitory nucleic acid targeting an immune checkpoint molecule, wherein the immune checkpoint molecule is programmed cell death 1 ligand 1 (PD-L1), that is covalently linked to the nanoparticle.

In some embodiments, the nucleic acid comprises a sequence of at least 10 contiguous nucleotides complementary to SEQ ID NO:1.

In some embodiments, the nucleic acid comprises at least one modified nucleotide, wherein the at least one modified nucleotide comprises at least one modification in its base and/or at least one modification in its sugar, or wherein the at least one modified nucleotide comprises a modification in an atom that forms a phosphodiester bond between two adjoining nucleotides in the nucleic acid.

In some embodiments, the at least one modified nucleotide is a locked nucleotide.

In some embodiments, the nucleic acid is a small interfering RNA (siRNA) molecule.

In some embodiments, the nucleic acid is covalently-linked to the nanoparticle through a chemical moiety comprising a disulfide bond or a thioether bond.

In some embodiments, the nanoparticle is magnetic.

In accordance with the invention, provided herein are pharmaceutical compositions comprising the therapeutic nanoparticles described herein, and optionally a chemotherapeutic agent, optionally wherein the chemotherapeutic agent is gemcitabine.

In accordance with the invention, provided are the therapeutic nanoparticles described herein in combination with a chemotherapeutic agent, for use in treating cancer in a subject.

In some embodiments, the cancer is selected from the group consisting of: breast cancer, colon cancer, kidney cancer, lung cancer, skin cancer, ovarian cancer, pancreatic cancer, prostate cancer, rectal cancer, stomach cancer, thyroid cancer, and uterine cancer.

In some embodiments, the methods include imaging a tissue of the subject to determine a location or number of cancer cells in the subject, or a location of the therapeutic nanoparticles in the subject.

In some embodiments, the therapeutic nanoparticle is administered in two or more doses to the subject, and optionally wherein the therapeutic nanoparticle is administered to the subject at least once a week.

In some embodiments, the therapeutic nanoparticle is administered to the subject by intravenous, subcutaneous, intraarterial, intramuscular, or intraperitoneal administration.

In some embodiments, the subject has pancreatic cancer.

In some embodiments, the chemotherapeutic agent is gemcitabine.

The term "magnetic" is used to describe a composition that is responsive to a magnetic field. Magnetic compositions (any of the therapeutic nanoparticles described herein) contain a material that is paramagnetic, superparamagnetic, or ferromagnetic. Magnetic compositions contain a metal oxide selected from the group of Fe(II) oxides.

The term "paramagnetic" is used to describe a composition that develops a magnetic moment only in the presence of an externally-applied magnetic field.

The term "ferromagnetic" or "ferromagnetic" is used to describe a composition that is strongly susceptible to magnetic fields and is capable of retaining magnetic properties (a magnetic moment) after an externally-applied magnetic field has been removed.

By the term "nanoparticle" is meant an object that has a diameter between 10 nm and 30 nm, between 10 nm and 20 nm, between 10 nm and 15 nm. Nanoparticles include the therapeutic nanoparticles described herein.

By the term "magnetic nanoparticle" is meant a nanoparticle ( any of the therapeutic nanoparticles described herein) that is magnetic (as defined herein). Magnetic nanoparticles are described herein.

By the term "polymer coating" is meant at least one molecular layer (e.g., homogenous or non-homogenous) of at least one polymer, dextran, applied to a surface of a three-dimensional object (e.g., a three-dimensional object containing a magnetic material, such as a metal oxide). Methods for applying a polymer coating to an object (e.g., a three-dimensional object containing a magnetic material) are described herein and are also known in the art.

By the term "nucleic acid" is meant any single- or double-stranded polynucleotide (e.g., DNA or RNA, cDNA, semi-synthetic, or synthetic origin). The term nucleic acid includes oligonucleotides containing at least one modified nucleotide (e.g., containing a modification in the base and/or a modification in the sugar) and/or a modification in the phosphodiester bond linking two nucleotides. In some embodiments, the nucleic acid can contain at least one locked nucleotide (LNA). Non-limiting examples of nucleic acids are described herein. Additional examples of nucleic acids are known in the art.

By the term "modified nucleotide" is meant a DNA or RNA nucleotide that contains at least one modification in its base and/or at least one modification in its sugar (ribose or deoxyribose). A modified nucleotide can also contain modification in an atom that forms a phosphodiester bond between two adjoining nucleotides in a nucleic acid sequence.

By the term "fluorophore" is meant a molecule that absorbs light at a first wavelength and emits light at a second wavelength, where the first wavelength is shorter (higher energy) than the second wavelength. In some embodiments, the first wavelength absorbed by the fluorophore can be in the near-infrared range. Non-limiting examples of fluorophores are described herein. Additional examples of fluorophores are known in the art.

By the term "near-infrared light" is meant light with a wavelength of between about 600 nm to about 3,000 nm.

By the term "targeting peptide" is meant a peptide that is bound by a molecule (e.g., protein, sugar, or lipid, or combination thereof) present in or on the plasma membrane of a target cell (e.g., a cancer cell). As described herein, a targeting peptide can be covalently linked to a secondary molecule or composition (e.g., any of the therapeutic nanoparticles described herein) to target the secondary molecule or composition to a target cell (e.g., a cancer cell). In some embodiments, a targeting peptide that is covalently linked to a secondary molecule or composition (e.g., any of the therapeutic nanoparticles described herein) results in the uptake of the secondary molecule or composition by the targeted cell (e.g., cellular uptake by endocytosis or pinocytosis). Non-limiting examples of targeting peptides are described herein. Additional examples of targeting peptides are known in the art.

By the term "small interfering RNA" or "siRNA" is meant a double-stranded nucleic acid molecule that is capable of mediating RNA interference in a cell. The process of RNA interference is described in Ebalshir et al. (Nature 411:494-498, 2001). Each strand of a siRNA can be between 19 and 23 nucleotides in length. As used herein, siRNA molecules need not be limited to those molecules containing only native or endogenous RNA nucleotides, but can further encompass chemically-modified nucleotides. Non-limiting examples of siRNA are described herein. Additional examples of siRNA are known in the art.

By the phrase "tumor growth" is meant the expansion of tumor mass in a subject. Non-limiting examples of tumor growth include: the formation of new tumor cells, the proliferation of existing tumor cells, the resistance to apoptosis in existing tumor cells. Exemplary methods for detecting and determining tumor growth are described herein. Additional methods for detecting and determining tumor growth are known in the art.

By the term "metastasis" is meant the migration of a cancer cell present in a primary tumor to a secondary, non-adjacent tissue in a subject. Non-limiting examples of metastasis include: metastasis from a primary tumor to a lymph node (e.g., a regional lymph node), bone tissue, lung tissue, liver tissue, and/or brain tissue. The term metastasis also includes the migration of a metastatic cancer cell found in a lymph node to a secondary tissue (e.g., bone tissue, liver tissue, or brain tissue). In some non-limiting embodiments, the cancer cell present in a primary tumor is a breast cancer cell, a colon cancer cell, a kidney cancer cell, a lung cancer cell, a skin cancer cell, an ovarian cancer cell, a pancreatic cancer cell, a prostate cancer cell, a rectal cancer cell, a stomach cancer cell, a thyroid cancer cell, or a uterine cancer cell. Additional aspects and examples of metastasis are known in the art or described herein.

By the term "primary tumor" is meant a tumor present at the anatomical site where tumor progression began and proceeded to yield a cancerous mass. In some embodiments, a physician may not be able to clearly identify the site of the primary tumor in a subject.

By the term "metastatic tumor" is meant a tumor in a subject that originated from a tumor cell that metastasized from a primary tumor in the subject. In some embodiments, a physician may not be able to clearly identify the site of the primary tumor in a subject.

By the term "lymph node" is meant a small spherical or oval-shaped organ of the immune system that contains a variety of cells including B-lymphocytes, T-lymphocytes, and macrophages, which is connected to the lymphatic system by lymph vessels. A variety of lymph nodes are present in a mammal including, but not limited to: axillary lymph nodes (e.g., lateral glands, anterior or pectoral glands, posterior or subscapular glands, central or intermediate glands, or medial or subclavicular glands), sentinel lymph nodes, sub-mandibular lymph nodes, anterior cervical lymph nodes, posterior cervical lymph nodes, supraclavicular lymph nodes, sub-mental lymph nodes, femoral lymph nodes, mesenteric lymph nodes, mediastinal lymph nodes, inguinal lymph nodes, subsegmental lymph nodes, segmental lymph nodes, lobar lymph nodes, interlobar lymph nodes, hilar lymph nodes, supratrochlear glands, deltoideopectoral glands, superficial inguinal lymph nodes, deep inguinal lymph nodes, brachial lymph nodes, and popliteal lymph nodes.

By the term "imaging" is meant the visualization of at least one tissue of a subject using a biophysical technique (e.g., electromagentic energy absorption and/or emission). Non-limiting embodiments of imaging include: magnetic resonance imaging (MRI), X-ray computed tomography, and optical imaging.

By the phrase "stabilization of tumor size" is meant that a tumor has reached a stage in which there is only an insignificant or non-detectable change in the total or approximate volume of a tumor in a subject over time.

By the phrase "rate of tumor growth" is meant a change in the total or approximate volume of a tumor or a change in the total or approximate number of cells present in a tumor over time in a subject. The rate of tumor growth can be determined using the exemplary methods described herein. Additional methods for determining the rate of tumor growth are known in the art.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. **In** case of conflict between the present specification and a reference mentioned herein, the present specification, including definitions, will control.

Other features and advantages of the invention will be apparent from the following detailed description and figures, and from the claims.

### DESCRIPTION OF DRAWINGS

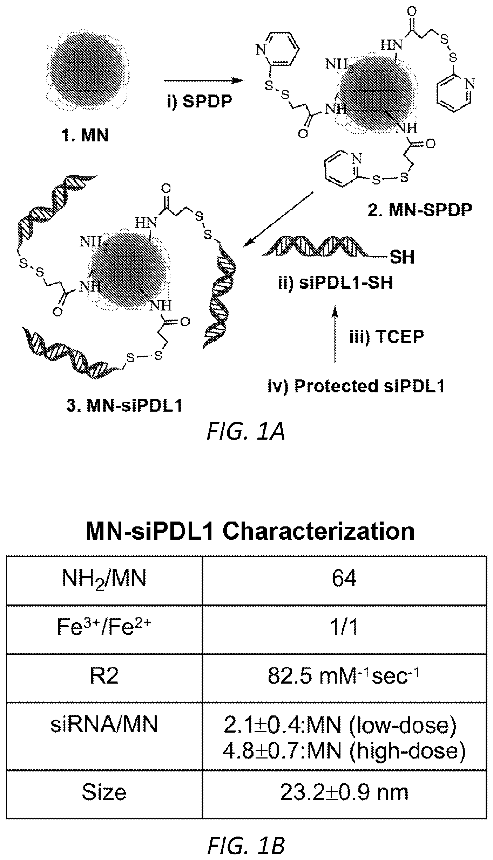

FIGs. 1A-B. Structure, synthesis, and characterization of exemplary MN-siPDL1. A. MN-siPDL1was synthesized by sequential conjugation of 20-nm aminated dextran-coated superparamagnetic iron oxide nanoparticles to the heterobifunctional labile linker SPDP and siRNA against PD-L1. B. MN-siPDL1 characterization.

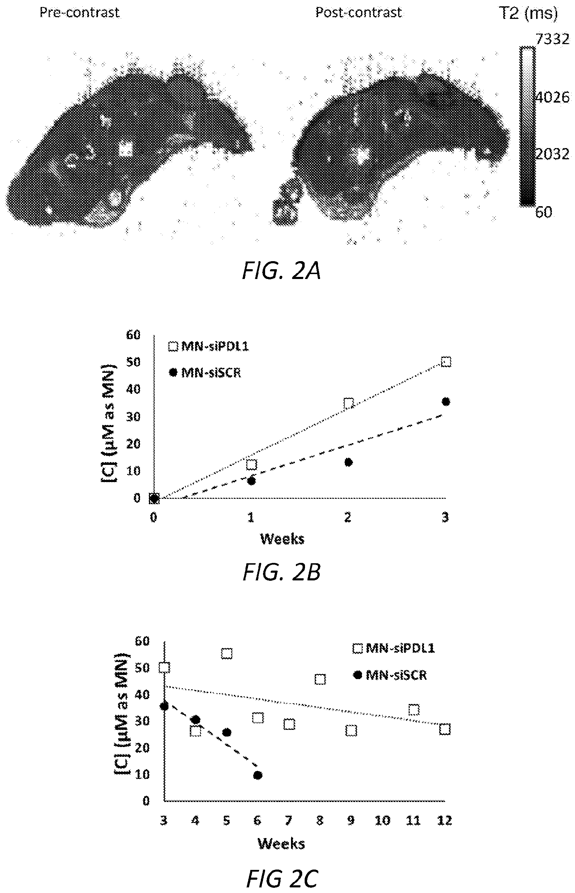

FIGs. 2A-C. Image-guided delivery of MN-siRNA to tumors. A. T2 maps of tumor bearing animals. The localization of MN-siPDL1 in tumor tissue caused shortening of the T2 relaxation time and resulted in negative contrast as compared to the pre-contrast image. B. MN-siPDL1 concentration measurements over the tumor region-of-interest (ROI) in experimental and control animals during the first 3 weeks of treatment. The accumulation rate of MN-siPDL1 was 1.5-fold faster than that of MN-siSCR during the first three weeks of treatment. C. MN-siPDL1 concentration measurements over the tumor region-of-interest (ROI) in experimental and control animals during weeks 3-12 of treatment. The concentration of the agent in the control group treated with MN-siSCR decreased 5.1-fold faster than in the experimental group treated with MN-siPDL1.

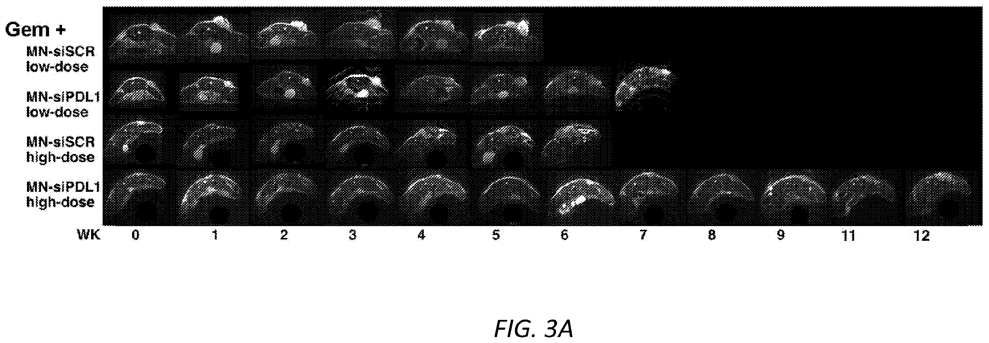

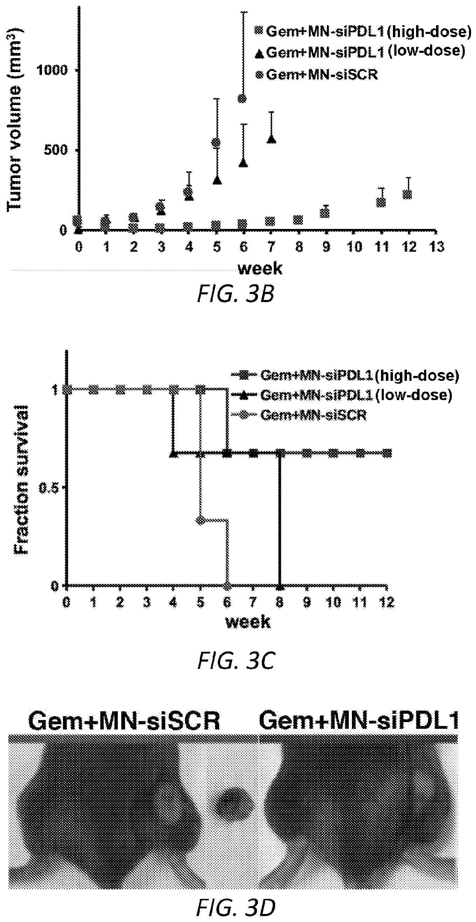

FIGs. 3A-D. Combination treatment with gemcitabine and MN-siPDL1. A. Representative color-coded T2-weighted MR images during the course of treatment. B. Change in tumor volume during treatment. The response was significantly different between the high-dose active MN-siPDL1and inactive MN-siSCR therapeutic in combination with gemcitabine beginning as early as week 2. In the low-dose MN-siPDL1 group, this difference was evident after week 6. C. Kaplan-Meier survival analysis demonstrating improvement in survival in animals treated with MN-siPDL1+gemcitabine vs. MN-siSCR+gemcitabine. D. Photographs of tumor-bearing mice at week 6 demonstrating necrosis and ulceration in the control tumors.

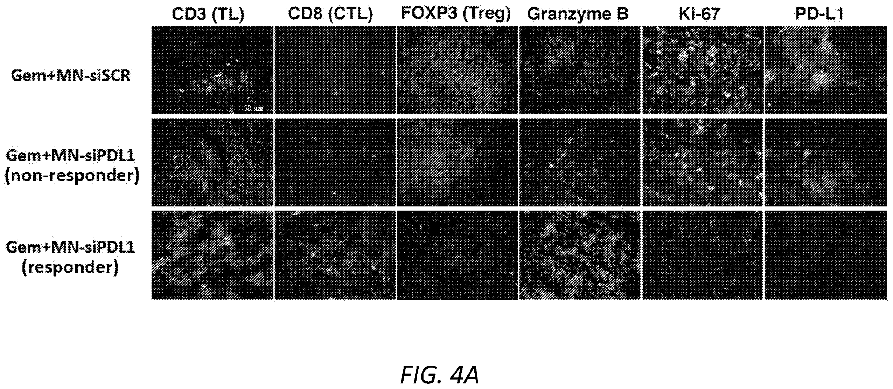

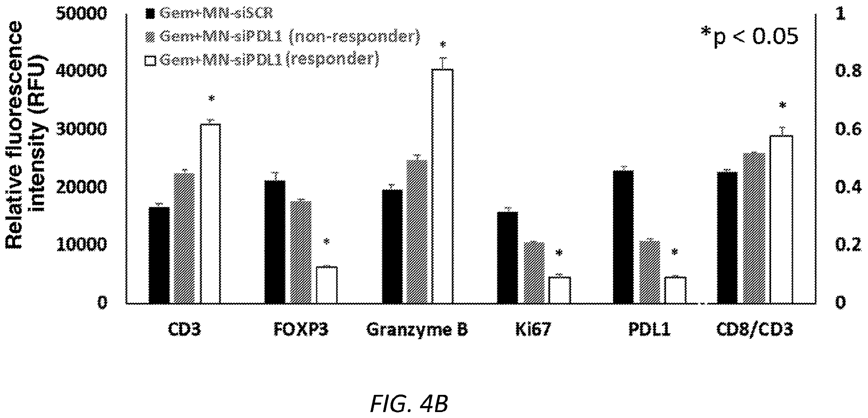

FIGs. 4A-B. Immunofluorescence of tumors from mice treated with MN-siPDL1 and gemcitabine. A. Representative micrographs and B. Quantitative analysis of fluorescence signal intensity demonstrating efficient PD-L1 inhibition, TIL recruitment and activation, Treg attenuation, and inhibition of tumor cell proliferation. TL: T lymphocytes; CTL: cytotoxic T lymphocytes; Treg: regulatory T cells; Ki-67: proliferation.

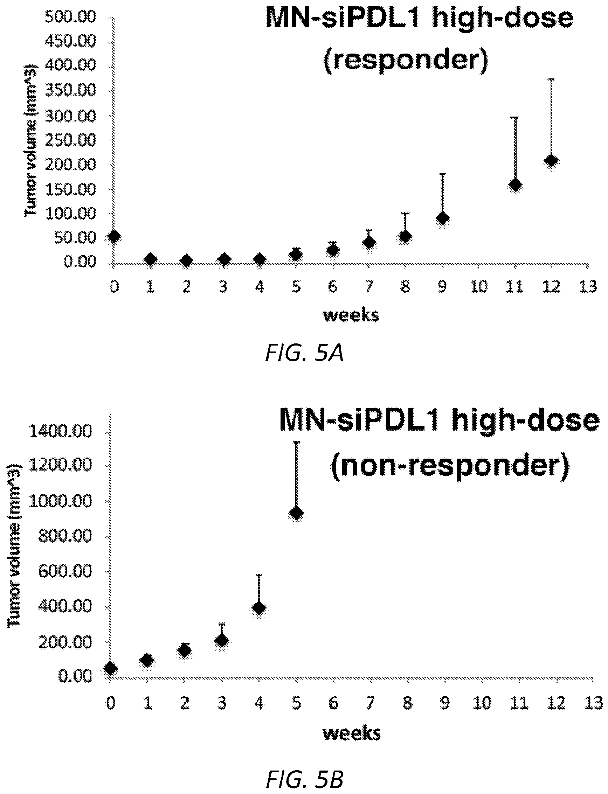

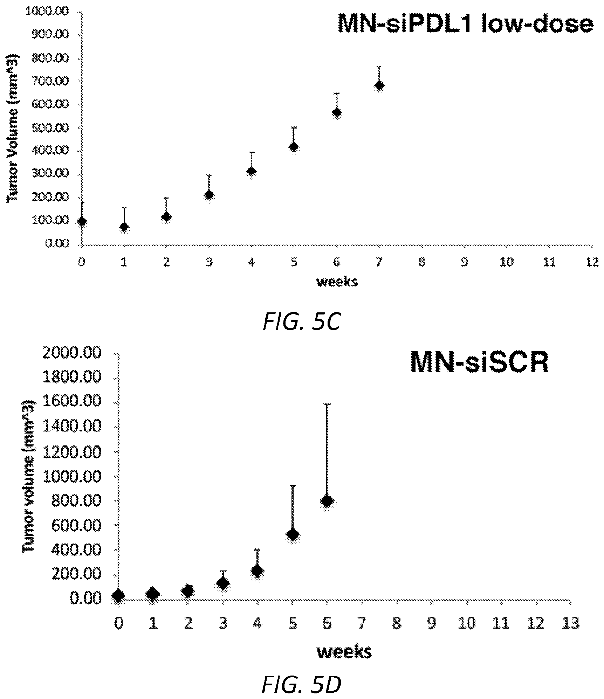

FIGs. 5A-D. Combination treatment with gemcitabine and MN-siPDL1. Change in tumor volume during treatment for each of the treatment groups. A, MN-siPDL1 high dose responders; B, MN-siPDL1high dose non-responders; C, MN-siPDL1 low dose; D, MN-siSCR (scrambled control). The sample size for the study was 6. In the high dose group, there were 2 mice that did not respond, i.e., their tumor volumes did not regress; their results are shown in Fig. 5B.

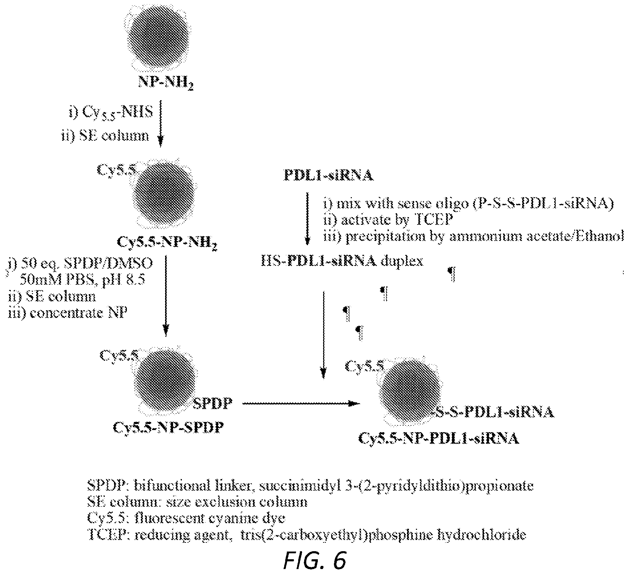

FIG. 6. Schematic with additional details of preparation of exemplary nanodrug (MN-siPDL1) for the inhibition of PD-L1 mRNA in cancer cells.

### DETAILED DESCRIPTION

While they hold great promise, clinical results with checkpoint inhibitors have demonstrated variability in the response. Pancreatic cancer, in particular, has proven resistant to initial immunotherapy approaches.

Pancreatic cancer is the fourth-leading cause of cancer-related death in the United States with an overall 5-year survival rate of only 8%.¹ Surgical resection remains the treatment of choice for patients with resectable disease. However, less than 20% of the diagnosed patients qualify for curative resections,² 30% of patients present with regional disease, and 50% present with distal metastases³ with survival rates of 11% and 2%, respectively.¹ The reasons behind such poor prognosis have been postulated to involve the advanced stage at the time of diagnosis,² and resistance to standard chemotherapies.⁴ There are multiple factors that are conceived to confer chemo-resistance: the formation of desmoplastic stroma limiting drug delivery, the activation of pancreatic stellate cells by reactive oxygen species, cytokines, and/or growth factors, and activated stellate cell secretion of immunosuppressive signaling molecules.^{4,5} Due to the complex tumor biology of pancreatic cancer, multiple combination chemotherapies have emerged. As such, FOLFIRINOX (a combination consisting of 5-fluorouracil, leucovorin, irinotecan, and oxaliplatin), and gemcitabine/nab-paclitaxel have shown improvements in overall survival compared to standard gemcitabine monotherapy treatment.^{6,7} However, these combination therapies are heavily dependent on the patient's overall health, and the overall survival benefit for the latest cytotoxic combination therapies is only ~ 2- 5 months.

In pancreatic cancer, advances in checkpoint inhibitor-based therapies have shown disappointing clinical results. In a Phase II trial anti-CTLA-4, Ipilimumab, monotherapy was ineffective with no responders resulting from the trial.²⁷ Similarly, in a multicenter Phase I trial an anti-PDL-1 antibody was administered intravenously in a variety of advanced cancer patients. Out of the 14 pancreatic cancer patients recruited there were no objective responses reported.²⁸

In light of the tremendous suffering caused by this disease and the modest progress achieved thus far with cytotoxic treatments, it is clear that we need to explore radical, transformative approaches for therapy that attack the disease from multiple angles.

The last decade has seen tremendous progress in the field of cancer immunotherapy. In fact, immunotherapy represents the most promising new cancer treatment approach since the development of the first chemotherapies in the 1940s. Checkpoint inhibitors have worked against lethal cancers such as melanoma and some lung cancers - sometimes with dramatic success - and are being tested in dozens of other cancer types.^{8,9} But pancreatic cancer has proven difficult to treat with conventional drugs and has been resistant to initial immunotherapy approaches. Partly, the reason for this is the complex tumor microenvironment that characterizes pancreatic adenocarcinoma. Chiefly, the presence of desmoplastic tumor stroma that is both immunosuppressive in nature and a physical barrier for antibody and T lymphocytes infiltration.¹⁰ Consequently, it is important to design alternative approaches that combine:
innovative checkpoint inhibitors that can be delivered efficiently to tumor cells and tumor resident macrophages, and strategies that enhance the permeation of the tumor by T lymphocytes.

Presented herein is an alternative strategy that relies on combining chemotherapeutics, e.g., gemcitabine (Gem), 5-FLOROURACIL, FOLFIRINOX, and a novel immune checkpoint inhibitor, PD-L1 inhibitor (termed MN-siPDL1), that incorporate a nanoparticle carrier that is delivered with high efficiency to tumor cells in vivo ¹¹⁻¹⁹, where it post-transcriptionally inhibits immune checkpoint molecules, PD-L1, expression on tumor cells via the RNA interference mechanism. The approach is advantageous over small molecules or antibodies because the siRNA component inhibits the target antigen at the post-transcriptional level and not at the protein level. Also, the RNAi mechanism is catalytic and necessitates the delivery of only picomolar amounts of siRNA to the tumor cell for the abolition of the target antigen. By contrast, small molecules or antibodies require the achievement of at least a 1:1 molar ratio of antigen to therapeutic molecule and could be ineffective in the presence of a compensatory increase in the expression of the target antigen by the tumor cell.

In the current study, 7 weeks of combination therapy consisting of gemcitabine and MN-siPDL1were administered in a syngeneic murine pancreatic cancer model. This approach resulted in significantly lower morbidity and toxicity, leading to tumor regression and a dramatic improvement in survival. In particular, following dose optimization, a 90% reduction in tumor volume was achieved 3 weeks after the beginning of treatment. Whereas 100% of the control animals had succumbed to their tumors by week 6 after the beginning of treatment, there was no mortality in the experimental group by week 5, and 67% of the experimental animals survived for 12 weeks.

The described methodology represents an integrated tool for drug delivery, image guidance of the delivery process, and a synchronous biomarker of therapeutic response.

### Compositions

Provided herein are therapeutic nanoparticles that have a diameter of between about 10 nm to about 30 nm, between about 10 nm to about 25 nm, between about 15 nm to about 25 nm, between about 20 nm and about 25 nm, and contain a polymer coating comprising dextran, and a nucleic acid containing at least 10 (e.g., at least 11, 12, 13, 14, 15, 16, 17, 18, 19, 20, or 21) contiguous nucleotides within a sequence that is complementary to a human immune checkpoint molecule, PD-L1.

### PD-L1

PD-L1 is also known as CD274, B7-H; B7H1; PDL1; PDCD1L1; and PDCD1LG1. Exemplary sequences for human PD-L1 are provided at the NCBI GenBank Acc. Nos. shown in Table A.

**Table A- Exemplary sequences for human PD-L1**

| Nucleic Acid | Notes | Protein | Notes |
|---|---|---|---|
| NM_014143.4 | Variant 1 | NP_054862.1 | isoform a precursor |
| NM_001267706.1 | Variant 2 | NP_001254635.1 | isoform b precursor |
| NM_001314029.1 | Variant 4 | NP_001300958.1 | isoform c precursor |

According to the NCBI reference notes, Variant 1 is the longest transcript and encodes the longest isoform (isoform a precursor). Variant 2 lacks an alternate in-frame exon in the 5' coding region, compared to variant 1, resulting in the shorter protein isoform b. Variant 4 lacks several exons and its 3' terminal exon extends past a splice site that is used in variant 1, resulting in a novel 3' coding region and novel 3' UTR compared to variant 1, and encodes isoform c (which is shorter than and has a distinct C-terminus compared to isoform a).

In the present methods and compositions, siRNA targeting any or all of the above (e.g., targeting a region that is common to all three of the above) can be used. The sequence of the human variant 1 mRNA is as follows:

1 agttctgcgc agcttcccga ggctccgcac cagccgcgct tctgtccgcc tgcagggcat
61 tccagaaaga tgaggatatt tgctgtcttt atattcatga cctactggca tttgctgaac
121 gcatttactg tcacggttcc caaggaccta tatgtggtag agtatggtag caatatgaca
181 attgaatgca aattcccagt agaaaaacaa ttagacctgg ctgcactaat tgtctattgg
241 gaaatggagg ataagaacat tattcaattt gtgcatggag aggaagacct gaaggttcag
301 catagtagct acagacagag ggcccggctg ttgaaggacc agctctccct gggaaatgct
361 gcacttcaga tcacagatgt gaaattgcag gatgcagggg tgtaccgctg catgatcagc
421 tatggtggtg ccgactacaa gcgaattact gtgaaagtca atgccccata caacaaaatc
481 aaccaaagaa ttttggttgt ggatccagtc acctctgaac atgaactgac atgtcaggct
541 gagggctacc ccaaggccga agtcatctgg acaagcagtg accatcaagt cctgagtggt
601 aagaccacca ccaccaattc caagagagag gagaagcttt tcaatgtgac cagcacactg
661 agaatcaaca caacaactaa tgagattttc tactgcactt ttaggagatt agatcctgag
721 gaaaaccata cagctgaatt ggtcatccca gaactacctc tggcacatcc tccaaatgaa
781 aggactcact tggtaattct gggagccatc ttattatgcc ttggtgtagc actgacattc
841 atcttccgtt taagaaaagg gagaatgatg gatgtgaaaa aatgtggcat ccaagataca
901 aactcaaaga agcaaagtga tacacatttg gaggagacgt aatccagcat tggaacttct
961 gatcttcaag cagggattct caacctgtgg tttaggggtt catcggggct gagcgtgaca
1021 agaggaagga atgggcccgt gggatgcagg caatgtggga cttaaaaggc ccaagcactg

1081 aaaatggaac ctggcgaaag cagaggagga gaatgaagaa agatggagtc aaacagggag
1141 cctggaggga gaccttgata ctttcaaatg cctgaggggc tcatcgacgc ctgtgacagg
1201 gagaaaggat acttctgaac aaggagcctc caagcaaatc atccattgct catcctagga
1261 agacgggttg agaatcccta atttgagggt cagttcctgc agaagtgccc tttgcctcca
1321 ctcaatgcct caatttgttt tctgcatgac tgagagtctc agtgttggaa cgggacagta
1381 tttatgtatg agtttttcct atttattttg agtctgtgag gtcttcttgt catgtgagtg
1441 tggttgtgaa tgatttcttt tgaagatata ttgtagtaga tgttacaatt ttgtcgccaa
1501 actaaacttg ctgcttaatg atttgctcac atctagtaaa acatggagta tttgtaaggt
1561 gcttggtctc ctctataact acaagtatac attggaagca taaagatcaa accgttggtt
1621 gcataggatg tcacctttat ttaacccatt aatactctgg ttgacctaat cttattctca
1681 gacctcaagt gtctgtgcag tatctgttcc atttaaatat cagctttaca attatgtggt
1741 agcctacaca cataatctca tttcatcgct gtaaccaccc tgttgtgata accactatta
1801 ttttacccat cgtacagctg aggaagcaaa cagattaagt aacttgccca aaccagtaaa
1861 tagcagacct cagactgcca cccactgtcc ttttataata caatttacag ctatatttta
1921 ctttaagcaa ttcttttatt caaaaaccat ttattaagtg cccttgcaat atcaatcgct
1981 gtgccaggca ttgaatctac agatgtgagc aagacaaagt acctgtcctc aaggagctca
2041 tagtataatg aggagattaa caagaaaatg tattattaca atttagtcca gtgtcatagc
2101 ataaggatga tgcgagggga aaacccgagc agtgttgcca agaggaggaa ataggccaat
2161 gtggtctggg acggttggat atacttaaac atcttaataa tcagagtaat tttcatttac
2221 aaagagaggt cggtacttaa aataaccctg aaaaataaca ctggaattcc ttttctagca
2281 ttatatttat tcctgatttg cctttgccat ataatctaat gcttgtttat atagtgtctg
2341 gtattgttta acagttctgt cttttctatt taaatgccac taaattttaa attcatacct
2401 ttccatgatt caaaattcaa aagatcccat gggagatggt tggaaaatct ccacttcatc
2461 ctccaagcca ttcaagtttc ctttccagaa gcaactgcta ctgcctttca ttcatatgtt
2521 cttctaaaga tagtctacat ttggaaatgt atgttaaaag cacgtatttt taaaattttt
2581 ttcctaaata gtaacacatt gtatgtctgc tgtgtacttt gctattttta tttattttag
2641 tgtttcttat atagcagatg gaatgaattt gaagttccca gggctgagga tccatgcctt
2701 ctttgtttct aagttatctt tcccatagct tttcattatc tttcatatga tccagtatat
2761 gttaaatatg tcctacatat acatttagac aaccaccatt tgttaagtat ttgctctagg
2821 acagagtttg gatttgttta tgtttgctca aaaggagacc catggctctc ccagggtgca
2881 ctgagtcaat ctagtcctaa aaagcaatct tattattaac tctgtatgac agaatcatgt
2941 ctggaacttt tgttttctgc tttctgtcaa gtataaactt cactttgatg ctgtacttgc
3001 aaaatcacat tttctttctg gaaattccgg cagtgtacct tgactgctag ctaccctgtg
3061 ccagaaaagc ctcattcgtt gtgcttgaac ccttgaatgc caccagctgt catcactaca
3121 cagccctcct aagaggcttc ctggaggttt cgagattcag atgccctggg agatcccaga
3181 gtttcctttc cctcttggcc atattctggt gtcaatgaca aggagtacct tggctttgcc
3241 acatgtcaag gctgaagaaa cagtgtctcc aacagagctc cttgtgttat ctgtttgtac
3301 atgtgcattt gtacagtaat tggtgtgaca gtgttctttg tgtgaattac aggcaagaat
3361 tgtggctgag caaggcacat agtctactca gtctattcct aagtcctaac tcctccttgt
3421 ggtgttggat ttgtaaggca ctttatccct tttgtctcat gtttcatcgt aaatggcata
3481 ggcagagatg atacctaatt ctgcatttga ttgtcacttt ttgtacctgc attaatttaa
3541 taaaatattc ttatttattt tgttacttgg tacaccagca tgtccatttt cttgtttatt
3601 ttgtgtttaa taaaatgttc agtttaacat ccca (SEQ ID
NO:1)

**Table B - Exemplary sequences for other human immune checkpoint molecules (not part of the invention)**

| immune checkpoint molecule | Nucleic Acid NCBI RefSeq ID | Notes | Protein NCBI RefSeq ID | Notes |
|---|---|---|---|---|
| PD-1 | NM_005018.3 | | NP_005009.2 | |
| CD40 | NM_001250.5 | Variant 1 | NP_001241.1 | Isoform 1 |
| | NM_152854.3 | Variant 2 | NP_690593.1 | Isoform 2 |
| | NM_001322422.1 | Variant 5 | NP_001309351.1 | Isoform 5 |
| | NM_001322421.1 | Variant 4 | NP_001309350.1 | Isoform 4 |
| | NM_001302753.1 | Variant 3 | NP_001289682.1 | Isoform 3 |
| | NM_001362758.1 | Variant 6 | NP_001349687.1 | Isoform 6 |
| CTLA-4 | NM_005214.5 | | NP_005205.2 | |
| Tim3 | NM_032782.5 | | NP_116171.3 | |
| Lag3 | NM_002286.6 | | NP_002277.4 | |
| TIGIT | NM_173799.4 | | NP_776160.2 | |
| B7-H3 | NM_001024736.2 | Variant 1 | NP_001019907.1 | Isoform a |
| | NM_001329628.1 | Variant 3 | NP_001316557.1 | Isoform b |
| | NM_001329629.1 | Variant 4 | NP_001316558.1 | Isoform c |
| | NM_025240.2 | Variant 2 | NP_079516.1 | Isoform b |
| VSIR/VISTA | NM_022153.2 | | NP_071436.1 | |
| VTCN1/ | NM_024626.4 | Variant 1 | NP_078902.2 | Isoform 1 |
| B7-H4 | NM_001253849.1 | Variant 2 | NP_001240778.1 | Isoform 2 |
| | NM_001253850.1 | Variant 3 | NP_001240779.1 | Isoform 3 |
| PVRIG | XM_011516575.2 | | XP_011514877.1 | |
| GARP | NM_005512.2 | Variant 1 | NP_005503.1 | Isoform 1 |
| | NM_001128922.2 | Variant 2 | NP_001122394.1 | Isoform 2 |
| BTLA | NM_181780.4 | Variant 1 | NP_861445.4 | Isoform 1 |
| | NM_001085357.1 | Variant 2 | NP_001078826.1 | Isoform 2 |

Although the present methods exemplify human subjects, other mammalian subjects, e.g., veterinary subjects such as cats, dogs, horses, pigs and sheep, can also be treated using the present methods. **In** preferred embodiments, the nucleic acid targets a sequence from the same species as the subject to be treated.

**In** some embodiments, the therapeutic nanoparticles provided herein can be spherical or ellipsoidal, or can have an amorphous shape. **In** some embodiments, the therapeutic nanoparticles provided herein can have a diameter (between any two points on the exterior surface of the therapeutic nanoparticle) of between about 10 nm to about 30 nm, between about 10 nm to about 25 nm, between about 15 nm to about 25 nm, or between about 20 nm to about 25 nm. Therapeutic nanoparticles having a diameter of between about 10 nm to about 30 nm localize to the lymph nodes in a subject.

In some embodiments, the compositions can contain a mixture of two or more of the different therapeutic nanoparticles described herein. In some embodiments, the compositions contain at least one therapeutic nanoparticle containing at least 10 contiguous nucleotides within the target sequence covalently linked to the nanoparticle (a nanoparticle for decrease miR-10b levels in a target cell), and at least one therapeutic nanoparticle containing a sequence that is complementary to a sequence of at least 10 other contiguous nucleotides present within a sequence,

The therapeutic nanoparticles are magnetic (e.g., contain a core of a magnetic material).

### Nanoparticles

In some embodiments, any of the therapeutic nanoparticles described herein can contain a core of a magnetic material (e.g., a therapeutic magnetic nanoparticle). In some embodiments, the magnetic material or particle can contain a diamagnetic, paramagnetic, superparamagnetic, or ferromagnetic material that is responsive to a magnetic field. Non-limiting examples of therapeutic magnetic nanoparticles contain a core of a magnetic material containing a metal oxide selected from the group of: magnetite; ferrites (e.g., ferrites of manganese, cobalt, and nickel); Fe(II) oxides, and hematite, and metal alloys thereof. The core of magnetic material can be formed by converting metal salts to metal oxides using methods known in the art (e.g., Kieslich et al., Inorg. Chem. 2011). In some embodiments, the nanoparticles contain cyclodextrin gold or quantum dots. Non-limiting examples of methods that can be used to generate therapeutic magnetic nanoparticles are described in Medarova et al., Methods Mol. Biol. 555:1-13, 2009; and Medarova et al., Nature Protocols 1:429-431, 2006. Additional magnetic materials and methods of making magnetic materials are known in the art. In some embodiments of the methods described herein, the position or localization of therapeutic magnetic nanoparticles can be imaged in a subject (e.g., imaged in a subject following the administration of one or more doses of a therapeutic magnetic nanoparticle).

In some embodiments, not part of the invention, the therapeutic nanoparticles described herein do not contain a magnetic material. In some embodiments, a therapeutic nanoparticle can contain, in part, a core of containing a polymer (e.g., poly(lactic-co-glycolic acid)). Skilled practitioners will appreciated that any number of art known materials can be used to prepare nanoparticles, including, but are not limited to, gums (e.g., Acacia, Guar), chitosan, gelatin, sodium alginate, and albumin. Additional polymers that can be used to generate the therapeutic nanoparticles described herein are known in the art. For example, polymers that can be used to generate the therapeutic nanoparticles include, but are not limited to, cellulosics, poly(2-hydroxy ethyl methacrylate), poly(N-vinyl pyrrolidone), poly(methyl methacrylate), poly(vinyl alcohol), poly(acrylic acid), polyacrylamide, poly(ethylene-co-vinyl acetate), poly(ethylene glycol), poly(methacrylic acid), polylactides (PLA), polyglycolides (PGA), poly(lactide-co-glycolides) (PLGA), polyanhydrides, polyorthoesters, polycyanoacrylate and polycaprolactone.

Skilled practitioners will appreciate that the material used in the composition of the nanoparticles, the methods for preparing, coating, and methods for controlling the size of the nanoparticles can vary substantially. However, these methods are well known to those in the art. Key issues include the biodegradability, toxicity profile, and pharmacokinetics/pharmacodynamics of the nanoparticles. The composition and/or size of the nanoparticles are key determinants of their biological fate. For example, larger nanoparticles are typically taken up and degraded by the liver, whereas smaller nanoparticles (<30 nm in diameter) typically circulate for a long time (sometimes over 24-hr blood half-life in humans) and accumulate in lymph nodes and the interstitium of organs with hyperpermeable vasculature, such as tumors.

### Polymer Coatings

The therapeutic nanoparticles described herein contain a polymer coating over the core magnetic material (e.g., over the surface of a magnetic material). The polymer material can be suitable for attaching or coupling one or more biological agents (e.g., such as any of the nucleic acids, fluorophores, or targeting peptides described herein). One of more biological agents (e.g., a nucleic acid, fluorophore, or targeting peptide) can be fixed to the polymer coating by chemical coupling (covalent bonds).

In some embodiments, the therapeutic nanoparticles are formed by a method that includes coating the core of magnetic material with a polymer that is relatively stable in water. In some embodiments, the therapeutic nanoparticles are formed by a method that includes coating a magnetic material with a polymer or absorbing the magnetic material into a thermoplastic polymer resin having reducing groups thereon. A coating can also be applied to a magnetic material using the methods described in U.S. Pat. Nos. 5,834,121, 5,395,688, 5,356,713, 5,318,797, 5,283,079, 5,232,789, 5,091,206, 4,965,007, 4,774,265, 4,770,183, 4,654,267, 4,554,088, 4,490,436, 4,336,173, and 4,421,660; and WO 10/111066.

Method for the synthesis of iron oxide nanoparticles include, for example, physical and chemical methods. For example, iron oxides can be prepared by coprecipitation of Fe2+ and Fe3+ salts in an aqueous solution. The resulting core consists of magnetite (Fe₃O₄), maghemite (γ-Fe₂O₃) or a mixture of the two. The anionic salt content (chlorides, nitrates, sulphates etc), the Fe2+ and Fe3+ ratio, pH and the ionic strength in the aqueous solution all play a role in controlling the size. It is important to prevent the oxidation of the synthesized nanoparticles and protect their magnetic properties by carrying out the reaction in an oxygen free environment under inert gas such as nitrogen or argon. The coating materials can be added during the coprecipitation process in order to prevent the agglomeration of the iron oxide nanoparticles into microparticles. Skilled practitioners will appreciated that any number of art known surface coating materials can be used for stabilizing iron oxide nanoparticles, among which are synthetic and natural polymers, such as, for example, dextran.

For example, U.S. Pat. No. 4,421,660 note that polymer coated particles of an inorganic material are conventionally prepared by (1) treating the inorganic solid with acid, a combination of acid and base, alcohol or a polymer solution; (2) dispersing an addition polymerizable monomer in an aqueous dispersion of a treated inorganic solid and (3) subjecting the resulting dispersion to emulsion polymerization conditions. (col. 1, lines 21-27) U.S. Pat. No. 4,421,660 also discloses a method for coating an inorganic nanoparticles with a polymer, which comprises the steps of (1) emulsifying a hydrophobic, emulsion polymerizable monomer in an aqueous colloidal dispersion of discrete particles of an inorganic solid and (2) subjecting the resulting emulsion to emulsion polymerization conditions to form a stable, fluid aqueous colloidal dispersion of the inorganic solid particles dispersed in a matrix of a water-insoluble polymer of the hydrophobic monomer (col. 1, lines 42-50).

Alternatively, polymer-coated magnetic material can be obtained commercially that meets the starting requirements of size. For example, commercially available ultrasmall superparamagnetic iron oxide nanoparticles include NC100150 Injection (Nycomed Amersham, Amersham Health) and Ferumoxytol (AMAG Pharmaceuticals, Inc.).

According to the invention, the polymer coating comprises dextran. In some embodiments, the polymer coating is dextran.

### Nucleic Acids

The therapeutic nanoparticles provided contain at least one nucleic acid comprising a sequence that is complementary at least 10 (e.g., at least 11, 12, 13, 14, 15, 16, 17, 18, 19, 20, 21, or 22) contiguous nucleotides within a sequence of an immune checkpoint molecule, a PD-L1 sequence, e.g., SEQ ID NO: 1, that is covalently-linked to the nanoparticle. In some embodiments, the covalently-linked nucleic acid molecule contains a sequence that is complementary to all or part of an mRNA encoding an immune checkpoint protein (e.g., any of the immune checkpoint proteins described herein). For example, the covalently-linked nucleic acid can be complementary to all or part of a non-coding region of the coding strand of a nucleotide sequence encoding an immune checkpoint protein (e.g., any of the immune checkpoint proteins described herein). Non-coding regions ("5' and 3' untranslated regions") are the 5' and 3' sequences that flank the coding region in a gene and are not translated into amino acids. In some embodiments, the nucleic acid covalently-linked to the therapeutic nanoparticle is complementary to the translational start codon or a sequence encoding amino acids 1 to 5 of an immune checkpoint protein (e.g., any of the immune checkpoint proteins described herein).

The attached nucleic acid can be single-stranded or double-stranded. In some embodiments, the nucleic acid has a total length of between 23 nucleotides and 50 nucleotides (e.g., between 23-30 nucleotides, between 30-40 nucleotides, and between 40-50 nucleotides). In some embodiments, the nucleic acid can be an antisense RNA or siRNA.

Antisense nucleic acid molecules can be covalently linked to the therapeutic nanoparticles described herein.

Based upon the sequences provided herein (e.g., the sequences for human immune checkpoint molecules, PD-L1, e.g., SEQ ID NO:1 and the other sequences in Tables A and B), one of skill in the art can easily choose and synthesize any of a number of appropriate antisense molecules (e.g., antisense molecules to target an immune checkpoint molecule, PD-L1). For example, an antisense nucleic acid that targets PD-L1 can contain a sequence complementary to at least 10 (e.g., at least 15 or 20) contiguous nucleotides present in SEQ ID NO: 1 or a sequence for PD-L1 known in the art.

An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or modified nucleotides (e.g., any of the modified oligonucleotides described herein) designed to increase the biological stability of the molecules or to increase the physical stability of the duplex formed between the antisense and sense nucleic acids, e.g., phosphorothioate derivatives and acridine-substituted nucleotides can be used. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest). In some embodiments, the antisense nucleic acid molecules described herein can hybridize to a target nucleic acid by conventional nucleotide complementarities and form a stable duplex.

An antisense nucleic acid molecule can be an α-anomeric nucleic acid molecule. An α-anomeric nucleic acid molecule forms specific double-stranded hybrids with complementary RNA in which, contrary to the usual β-units, the strands run parallel to each other (Gaultier et al., Nucleic Acids Res. 15:6625-6641, 1987). The antisense nucleic acid molecule can also comprise a 2'-O-methylribonucleotide (Inoue et al., Nucleic Acids Res., 15:6131-6148, 1987) or a chimeric RNA-DNA analog (Inoue et al., FEES Lett. 215:327-330, 1987).

In some embodiments, the nucleic acid is a small interfering RNA (siRNA). RNAi is a process in which RNA is degraded in host cells. To decrease expression of an RNA, double-stranded RNA (dsRNA) containing a sequence corresponding to a portion of the target RNA (e.g., an immune checkpoint molecule, e.g., human PD-L1) is introduced into a cell. The dsRNA is digested into 21-23 nucleotide-long duplexes called short interfering RNAs (or siRNAs), which bind to a nuclease complex to form what is known as the RNA-induced silencing complex (or RISC). The RISC targets the endogenous target RNA by base pairing interactions between one of the siRNA strands and the endogenous RNA. It then cleaves the endogenous RNA about 12 nucleotides from the 3' terminus of the siRNA (see Sharp et al., Genes Dev. 15:485-490, 2001, and Hammond et al., Nature Rev. Gen. 2:110-119, 2001).

Standard molecular biology techniques can be used to generate siRNAs. Short interfering RNAs can be chemically synthesized, recombinantly produced, e.g., by expressing RNA from a template DNA, such as a plasmid, or obtained from commercial vendors such as Dharmacon. The RNA used to mediate RNAi can include modified nucleotides (e.g., any of the modified nucleotides described herein), such as phosphorothioate nucleotides. The siRNA molecules used to decrease the levels of mature human miR-10b can vary in a number of ways. For example, they can include a 3' hydroxyl group and strands of 21, 22, or 23 consecutive nucleotides. They can be blunt ended or include an overhanging end at either the 3' end, the 5' end, or both ends. For example, at least one strand of the RNA molecule can have a 3' overhang from about 1 to about 6 nucleotides (e.g., 1-5, 1-3, 2-4 or 3-5 nucleotides (whether pyrimidine or purine nucleotides) in length. Where both strands include an overhang, the length of the overhangs may be the same or different for each strand. To further enhance the stability of the RNA duplexes, the 3' overhangs can be stabilized against degradation (by, e.g., including purine nucleotides, such as adenosine or guanosine nucleotides, or replacing pyrimidine nucleotides with modified nucleotides (e.g., substitution of uridine two-nucleotide 3' overhangs by 2'-deoxythymidine is tolerated and does not affect the efficiency of RNAi). Any siRNA can be used provided it has sufficient homology to the target of interest. There is no upper limit on the length of the siRNA that can be used (e.g., the siRNA can range from about 21-50, 50-100, 100-250, 250-500, or 500-1000 base pairs).

In some embodiments, the nucleic acid molecule can contain at least one modified nucleotide (a nucleotide containing a modified base or sugar). In some embodiments, the nucleic acid molecule can contain at least one modification in the phosphate (phosphodiester) backbone. The introduction of these modifications can increase the stability, or improve the hybridization or solubility of the nucleic acid molecule.

The molecules described herein can contain one or more (e.g., two, three, four, of five) modified nucleotides. The modified nucleotides can contain a modified base or a modified sugar. Non-limiting examples of modified bases include: 8-oxo-N⁶-methyladenine, 7-deazaxanthine, 7-deazaguanine, N⁴, N⁴-ethanocytosin, N⁶, N⁶-ethano-2,6-diaminopurine, 5-(C³-C⁶)-alkynyl-cytosine, pseudoisocytosine, 2-hydroxy-5-methyl-4-triazolopyridin, isocytosine, isoguanine, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

Additional non-limiting examples of modified bases include those nucleobases described in U.S. Pat. Nos. 5,432,272 and 3,687,808, Freier et al., Nucleic Acid Res. 25:4429-4443, 1997; Sanghvi, Antisense Research and Application, Chapter 15, Ed. S. T. Crooke and B. Lebleu, CRC Press, 1993; Englisch, et al., Angewandte Chemie 30:613-722, 1991, Kroschwitz, Concise Encyclopedia of Polymer Science and Engineering, John Wiley & Sons, pp. 858-859, 1990; and Cook, Anti-Cancer Drug Design 6:585-607, 1991. Additional non-limiting examples of modified bases include universal bases (e.g., 3-nitropyrole and 5-nitroindole). Other modified bases include pyrene and pyridyloxazole derivatives, pyrenyl, pyrenylmethylglycerol derivatives, and the like. Other preferred universal bases include pyrrole, diazole, or triazole derivatives, including those universal bases known in the art.

In some embodiments, the modified nucleotide can contain a modification in its sugar moiety. Non-limiting examples of modified nucleotides that contain a modified sugar are locked nucleotides (LNAs). LNA monomers are described in WO 99/14226 and U.S. Patent Application Publications Nos. 20110076675, 20100286044, 20100279895, 20100267018, 20100261175, 20100035968, 20090286753, 20090023594, 20080096191, 20030092905, 20020128381, and 20020115080. Additional non-limiting examples of LNAs are disclosed in U.S. Patent No. 6,043,060, U.S. Patent No. 6,268,490, WO 01/07455, WO 01/00641, WO 98/39352, WO 00/56746, WO 00/56748, and WO 00/66604, as well as in Morita et al., Bioorg. Med. Chem. Lett. 12(1):73-76, 2002; Hakansson et al., Bioorg. Med. Chem. Lett. 11(7):935-938, 2001; Koshkin et al., J. Org. Chem. 66(25):8504-8512, 2001; Kvaerno et al., J. Org. Chem. 66(16):5498-5503, 2001; Hakansson et al., J. Org. Chem. 65(17):5161-5166, 2000; Kvaerno et al., J. Org. Chem. 65(17):5167-5176, 2000; Pfundheller et al., Nucleosides Nucleotides 18(9):2017-2030, 1999; and Kumar et al., Bioorg. Med. Chem. Lett. 8(16):2219-2222, 1998. In some embodiments, the modified nucleotide is an oxy-LNA monomer, such as those described in WO 03/020739.

Modified nucleotides can also include antagomirs (2'-O-methyl-modified, cholesterol-conjugated single stranded RNA analogs); ALN ( -L-LNA); ADA (2'-N-adamantylmethylcarbonyl-2'-amino-LNA); PYR (2'-N-pyrenyl-1-methyl-2'-amino-LNA); OX (oxetane-LNA); ENA (2'-O, 4"-C-ethylene bridged nucleic acid); AENA (2'-deoxy-2'-N, 4'-C-ethylene-LNA); CLNA (2',4'-carbocyclic-LNA); and CENA (2',4'-carbocyclic-ENA); HM-modified DNAs (4'-C-hydroxymethyl-DNA); 2'-substituted RNAs (with 2'-O-methyl, 2'-fluoro, 2'-aminoethoxymethyl, 2'-aminopropoxymethyl, 2'-aminoethyl, 2'-guanidinoethyl, 2'-cyanoethyl, 2'-aminopropyl); and RNAs with radical modifications of the ribose sugar ring, such as Unlocked Nucleic Acid (UNA), Altritol Nucleic Acid (ANA) and Hexitol Nucleic Acid (HNA) (see, Bramsen et al., Nucleic Acids Res. 37:2867-81, 2009).

The molecules described herein can also contain a modification in the phosphodiester backbone. For example, at least one linkage between any two contiguous (adjoining) nucleotides in the molecule can be connected by a moiety containing 2 to 4 groups/atoms selected from the group of: -CH₂-, -O-, -S-, -NR^{H}-, >C=O, >C=NR^{H}, >C=S, -Si(R")₂-, -SO-, -S(O)₂-, -P(O)₂-, - PO(BH₃)-, -P(O,S)-, -P(S)₂-, -PO(R")-, -PO(OCH₃)-, and - PO(NHR^{H})-, where R^{H} is selected from hydrogen and C₁₋₄-alkyl, and R" is selected from C₁₋₆-alkyl and phenyl. Illustrative examples of such linkages are -CH₂-CH₂-CH₂-, -CH₂-CO-CH₂-, -CH₂-CHOH-CH₂-, -O-CH₂-O-, -O-CH₂-CH₂-, -O-CH₂-CH= (including R⁵ when used as a linkage to a succeeding monomer), -CH₂-CH₂-O-, -NR^{H}-CH₂-CH₂-, -CH₂-CH₂-NR^{H}-, -CH₂-NR^{H}-CH₂-, -O-CH₂-CH₂-NR^{H}-, -NR^{H}-CO-O-, - NR^{H}-CO-NR^{H}-, -NR^{H}-CS-NR^{H}-, -NR^{H}-C(=NR^{H})-NR^{H}-, -NR^{H}-CO-CH₂-NR^{H}-, -O-CO-O-, -O-CO-CH₂-O-, -O-CH₂-COO-, -CH₂-CO-NR^{H}-, -O-CO-NR^{H}-, -NR^{H}-CO-CH₂-, -O-CH₂-CO-NR^{H}-, -O-CH₂-CH₂-NR^{H}-, -CH=N-O-, -CH₂-NR^{H}-O-, -CH₂-O-N= (including R⁵ when used as a linkage to a succeeding monomer), -CH₂-O-NR^{H}-, -CO-NR^{H}-CH₂-, -CH₂-NR^{H}-O-, - CH₂-NR^{H}-CO-, -O-NR^{H}-CH₂-, -O-NR^{H}-, -O-CH₂-S-, -S-CH₂-O-, -CH₂-CH₂-S-, -O-CH₂-CH₂-S-, -S-CH₂-CH= (including R⁵ when used as a linkage to a succeeding monomer), -S-CH₂-CH₂-, -S-CH₂-CH₂-O-, -S-CH₂-CH₂-S-, -CH₂-S-CH₂-, -CH₂-SO-CH₂-, -CH₂-SO₂-CH₂-, -O-SO-O-, -O-S(O)₂-O-, -O-S(O)₂-CH₂-, -O-S(O)₂-NR^{H}-, -NR_{H}-S(O)₂-CH₂-, -O-S(O)₂-CH₂-, -O-P(O)₂-O-, -O-P(O,S)-O-, -O-P(S)₂-O-, -S-P(O)₂-O-, -S-P(O,S)-O-, -S-P(S)₂-O-, -O-P(O,S)-S-, -O-P(S)₂-S-, -S-P(O)₂-S-, -S-P(O,S)-S-, -S-P(S)₂-S-, -O-PO(R")-O-, - O-PO(OCH₃)-O-, -O-PO-(OCH₂CH₃)-O-, -O-PO(OCH₂S-R)-O-, -O-PO(BH₃)-O-, -O-PO(NHR^{N})-O-, -O-P(O)₂-NR^{H}-, -NR^{H}-P(O)₂-O-, -O-P(O,NR^{H})2-O-, -CH₂-P(O)₂-0-, -O-P(O)₂-CH₂-, and -O-Si(R")₂-O-; among which -CH₂-CO-NR^{H}-, -CH₂-NR^{H}-O-, -S-CH₂-O-, -O-P(O)₂-O-, -O-P(O,S)-O-, -O-P(S)₂-O-, - **NR^{H}-P(O)₂-O-, -O-P(O,NR^{H})-O-, -O-PO(R**")-O-, **-O-**PO(CH₃)-O-, and -O-PO(NHR^{N})-O-, where R^{H} is selected form hydrogen and C₁₋₄-alkyl, and R" is selected from C₁₋₆-alkyl and phenyl. Further illustrative examples are given in Mesmaeker et. al., Curr. Opin. Struct. Biol. 5:343-355, 1995; and Freier et al., Nucleic Acids Research 25:4429-43, 1997. The left-hand side of the inter-nucleoside linkage is bound to the 5-membered ring as substituent P* at the 3'-position, whereas the right-hand side is bound to the 5'-position of a preceding monomer.

In some embodiments, the deoxyribose phosphate backbone of the nucleic acid can be modified to generate peptide nucleic acids (see Hyrup et al., Bioorganic & Medicinal Chem. 4(1): 5-23, 1996). Peptide nucleic acids (PNAs) are nucleic acid mimics, e.g., DNA mimics, in which the deoxyribose phosphate backbone is replaced by a pseudopeptide backbone and only the four natural nucleobases are retained. The neutral backbone of PNAs allows for specific hybridization to DNA and RNA under conditions of low ionic strength. The synthesis of PNA oligomers can be performed using standard solid phase peptide synthesis protocols, e.g., as described in Hyrup et al., 1996, *supra;* Perry-O'Keefe et al., Proc. Natl. Acad. Sci. U.S.A. 93:14670-675, 1996.

PNAs can be modified, e.g., to enhance their stability or cellular uptake, by attaching lipophilic or other helper groups to PNA, by the formation of PNA-DNA chimeras, or by the use of liposomes or other techniques of delivery known in the art. For example, PNA-DNA chimeras can be generated which may combine the advantageous properties of PNA and DNA. Such chimeras allow DNA recognition enzymes, e.g., RNAse H, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup, 1996, *supra).* The synthesis of PNA-DNA chimeras can be performed as described in Hyrup, 1996, *supra,* and Finn et al., Nucleic Acids Res. 24:3357-63, 1996. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al., Nucleic Acids Res., 17:5973-88, 1989). PNA monomers are then coupled in a stepwise manner to produce a chimeric molecule with a 5' PNA segment and a 3' DNA segment (Finn et al., Nucleic Acids Res. 24:3357-63, 1996). Alternatively, chimeric molecules can be synthesized with a 5' DNA segment and a 3' PNA segment (Peterser et al., Bioorganic Med. Chem. Lett. 5:1119-11124, 1975).

In some embodiments, any of the nucleic acids described herein can be modified at either the 3' or 5' end (depending on how the nucleic acid is covalently-linked to the therapeutic nanoparticle) by any type of modification known in the art. For example, either end may be capped with a protecting group, attached to a flexible linking group, or attached to a reactive group to aid in attachment to the substrate surface (the polymer coating). Non-limiting examples of 3' or 5' blocking groups include: 2-amino-2-oxyethyl, 2-aminobenzoyl, 4-aminobenzoyl, acetyl, acetyloxy, (acetylamino)methyl, 3-(9-acridinyl), tricyclo[3.3.1.1(3,7)]dec-1-yloxy, 2-aminoethyl, propenyl, (9-anthracenylmethoxy)carbonyl, (1,1-dmimethylpropoxy)carbonyl, (1,1-dimethylpropoxy)carbonyl, [1-methyl-1-[4-(phenylazo)phenyl]ethoxy]carbonyl, bromoacetyl, (benzoylamino)methyl, (2-bromoethoxy)carbonyl, (diphenylmethoxy)carbonyl, 1-methyl-3-oxo-3-phenyl-1-propenyl, (3-bromo-2-nitrophenyl)thio, (1,1-dimethylethoxy)carbonyl, [[(1,1-dimethylethoxy)carbonyl] amino]ethyl, 2-(phenylmethoxy)phenoxy, (1=[1,1'-biphenyl]-4-yl-1-methylethoxy) carbonyl, bromo, (4-bromophenyl)sulfonyl, 1H-benzotriazol-1-yl, [(phenylmethyl) thio]carbonyl, [(phenylmetyl)thio]methyl, 2-methylpropyl, 1,1-dimethylethyl, benzoyl, diphenylmethyl, phenylmethyl, carboxyacetyl, aminocarbonyl, chlorodifluoroacetyl, trifluoromethyl, cyclohexylcarbonyl, cycloheptyl, cyclohexyl, cyclohexylacetyl, chloro, carboxymethyl, cyclopentylcarbonyl, cyclopentyl, cyclopropylmethyl, ethoxycarbonyl, ethyl, fluoro, formyl, 1-oxohexyl, iodo, methyl, 2-methoxy-2-oxoethyl, nitro, azido, phenyl, 2-carboxybenzoyl, 4-pyridinylmethyl, 2-piperidinyl, propyl, 1-methylethyl, sulfo, and ethenyl. Additional examples of 5' and 3' blocking groups are known in the art. In some embodiments, the 5' or 3' blocking groups prevent nuclease degradation of the molecule.

The nucleic acids described herein can be synthesized using any methods known in the art for synthesizing nucleic acids (see, e.g., Usman et al., J. Am. Chem. Soc. 109:7845, 1987; Scaringe et al., Nucleic Acid Res. 18:5433, 1990; Wincott et al., Methods Mol. Biol. 74:59, 1997; and Milligan, Nucleic Acid Res. 21:8783, 1987). These typically make use of common nucleic acid protecting and coupling groups. Synthesis can be performed on commercial equipment designed for this purpose, e.g., a 394 Applied Biosystems, Inc. synthesizer, using protocols supplied by the manufacturer. Additional methods for synthesizing the molecules described herein are known in the art. Alternatively, the nucleic acids can be specially ordered from commercial vendors that synthesize oligonucleotides.

In some embodiments, the nucleic acid is attached to the therapeutic nanoparticle at its 5' end. In some embodiments, the nucleic acid is attached to the therapeutic nanoparticle at its 3' end. In some embodiments, the nucleic acid is attached to the therapeutic nanoparticle through a base present in the nucleic acid.

In some embodiments, the nucleic acid (e.g., any of the nucleic acids described herein) is attached to the therapeutic nanoparticle (e.g., to the polymer coating of the therapeutic nanoparticle) through a chemical moiety that contains a thioether bond or a disulfide bond. In some embodiments, the nucleic acid is attached to the therapeutic nanoparticle through a chemical moiety that contains an amide bond. Additional chemical moieties that can be used to covalently link a nucleic acid to a therapeutic nanoparticle are known in the art.

A variety of different methods can be used to covalently link a nucleic acid to a therapeutic nanoparticle. Non-limiting examples of methods that can be used to link a nucleic acid to a magnetic particle are described in EP 0937097; US RE41005; Lund et al., Nucleic Acid Res. 16:10861, 1998; Todt et al., Methods Mol. Biol. 529:81-100, 2009; Brody et al., J. Biotechnol. 74:5-13, 2000; Ghosh et al., Nucleic Acids Res. 15:5353-5372, 1987; U.S. Patent No. 5,900,481; U.S. Patent No. 7,569,341; U.S. Patent No. 6,995,248; U.S. Patent No. 6,818,394; U.S. Patent No. 6,811,980; U.S. Patent No. 5,900,481; and U.S. Patent No. 4,818,681. In some embodiments, carboiimide is used for the end-attachment of a nucleic acid to a therapeutic nanoparticle. In some embodiments, the nucleic acid is attached to the therapeutic nanoparticle through the reaction of one of its bases with an activated moiety present on the surface of the therapeutic nanoparticle (e.g., the reaction of an electrophilic base with a nucleophilic moiety on the surface of the therapeutic nanoparticle, or the reaction of a nucleophilic base with a electrophilic residue on the surface of the therapeutic nanoparticle). In some embodiments, a 5'-NH₂ modified nucleic acid is attached to a therapeutic nanoparticle containing CNBr-activated hydroxyl groups (see, e.g., Lund et al., *supra).* Additional methods for attaching an amino-modified nucleic acid to a therapeutic nanoparticle are described below. In some embodiments, a 5'-phosphate nucleic acid is attached to a therapeutic nanoparticle containing hydroxyl groups in the presence of a carbodiimide (see, e.g., Lund et al., *supra).* Other methods of attaching a nucleic acid to a therapeutic nanoparticle include carboiimide-mediated attachment of a 5'-phosphate nucleic acid to a NH₂ group on a therapeutic nanoparticle, and carboiimide-mediated attachment of a 5'-NH₂ nucleic acid to a therapeutic nanoparticle having carboxyl groups (see, e.g., Lund et al., *supra).*

In exemplary methods, a nucleic acid can be produced that contains a reactive amine or a reactive thiol group. The amine or thiol in the nucleic acid can be linked to another reactive group. The two common strategies to perform this reaction are to link the nucleic acid to a similar reactive moiety (amine to amine or thiol to thiol), which is called homobifunctional linkage, or to link to the nucleic acid to an opposite group (amine to thiol or thiol to amine), known as heterobifunctional linkage. Both techniques can be used to attach a nucleic acid to a therapeutic nanoparticle (see, for example, Misra et al., Bioorg. Med. Chem. Lett. 18:5217-5221, 2008; Mirsa et al., Anal. Biochem. 369:248-255, 2007; Mirsa et al., Bioorg. Med. Chem. Lett. 17:3749-3753, 2007; and Choithani et al., Methods Mol Biol. 381:133-163, 2007).

Traditional attachment techniques, especially for amine groups, have relied upon homobifunctional linkages. One of the most common techniques has been the use of bisaldehydes such as glutaraldehyde. Disuccinimydyl suberate (DSS), commercialized by Syngene (Frederick, MD) as synthetic nucleic acid probe (SNAP) technology, or the reagent p-phenylene diisothiocyanate can also be used to generate a covalent linkage between the nucleic acid and the therapeutic nanoparticle. N,N'-o-phenylenedimaleimide can be used to cross-link thiol groups. With all of the homobifunctional cross-linking agents, the nucleic acid is initially activated and then added to the therapeutic nanoparticle (see, for example, Swami et al., Int. J. Pharm. 374:125-138, 2009, Todt et al., Methods Mol. Biol. 529:81-100, 2009; and Limanskiǐ, Biofizika 51:225-235, 2006).

Heterobifunctional linkers can also be used to attach a nucleic acid to a therapeutic nanoparticle. For example, N-succinidimidyl-3-(2-pyridyldithio)proprionate (SPDP) initially links to a primary amine to give a dithiol-modified compound. This can then react with a thiol to exchange the pyridylthiol with the incoming thiol (see, for example, Nostrum et al., J. Control Release 15;153(1):93-102, 2011, and Berthold et al., Bioconjug. Chem. 21:1933-1938, 2010).

An alternative approach for thiol use has been a thiol-exchange reaction. If a thiolated nucleic acid is introduced onto a disulfide therapeutic nanoparticle, a disulfide-exchange reaction can occur that leads to the nucleic acid being covalently bonded to the therapeutic nanoparticle by a disulfide bond. A multitude of potential cross-linking chemistries are available for the heterobifunctional cross-linking of amines and thiols. Generally, these procedures have been used with a thiolated nucleotide. Reagents typically employed have been NHS (N-hydroxysuccinimide ester), MBS (m-maleimidobenzoyl-N-succinimide ester), and SPDP (a pyridyldisulfide-based system). The heterobifunctional linkers commonly used rely upon an aminated nucleic acid. Additional methods for covalently linking a nucleic acid to a therapeutic nanoparticle are known in the art.

### Targeting peptide

In some embodiments, the therapeutic nanoparticle further contains a covalently-linked targeting peptide, e.g., as described in WO2013/016126. By the term "targeting peptide" is meant a peptide that is bound by a molecule (e.g., protein, sugar, or lipid, or combination thereof) present in or on the plasma membrane of a target cell (e.g., a cancer cell). As described herein, a targeting peptide can be covalently linked to a secondary molecule or composition (e.g., any of the therapeutic nanoparticles described herein) to target the secondary molecule or composition to a target cell (e.g., a cancer cell). In some embodiments, a targeting peptide that is covalently linked to a secondary molecule or composition (e.g., any of the therapeutic nanoparticles described herein) results in the uptake of the secondary molecule or composition by the targeted cell (e.g., cellular uptake by endocytosis or pinocytosis). Non-limiting examples of targeting peptides are described herein. In some embodiments, the targeting peptide contains an RGD peptide, an EPPT peptide, NYLHNHPYGTVG (SEQ ID NO: 2), SNPFSKPYGLTV (SEQ ID NO: 3), GLHESTFTQRRL (SEQ ID NO: 4), YPHYSLPGSSTL (SEQ ID NO: 5), SSLEPWHRTTSR (SEQ ID NO: 6), LPLALPRHNASV (SEQ ID NO: 7), or βAla-(Arg)7-Cys (SEQ ID NO: 8). In some embodiments, the targeting peptide is covalently linked to the nanoparticle through a chemical moiety that contains a disulfide bond. In some embodiments, the therapeutic nanoparticle is magnetic. Additional examples of targeting peptides, and methods for attaching them to the nanoparticle, are known in the art; see, e.g., WO2013/016126.

### Pharmaceutical Compositions

Also provided herein are pharmaceutical compositions that contain a therapeutic nanoparticle as described herein. Two or more (e.g., two, three, or four) of any of the types of therapeutic nanoparticles described herein can be present in a pharmaceutical composition in any combination. The pharmaceutical compositions can be formulated in any manner known in the art.

Pharmaceutical compositions are formulated to be compatible with their intended route of administration (e.g., intravenous, intraarterial, intramuscular, intradermal, subcutaneous, or intraperitoneal). The compositions can include a sterile diluent (e.g., sterile water or saline), a fixed oil, polyethylene glycol, glycerine, propylene glycol or other synthetic solvents, antibacterial or antifungal agents such as benzyl alcohol or methyl parabens, chlorobutanol, phenol, ascorbic acid, thimerosal, and the like, antioxidants such as ascorbic acid or sodium bisulfite, chelating agents such as ethylenediaminetetraacetic acid, buffers such as acetates, citrates, or phosphates, and isotonic agents such as sugars (e.g., dextrose), polyalcohols (e.g., manitol or sorbitol), or salts (e.g., sodium chloride), or any combination thereof. Liposomal suspensions can also be used as pharmaceutically acceptable carriers (see, e.g., U.S. Patent No. 4,522,811). Preparations of the compositions can be formulated and enclosed in ampules, disposable syringes, or multiple dose vials. Where required (as in, for example, injectable formulations), proper fluidity can be maintained by, for example, the use of a coating such as lecithin, or a surfactant. Absorption of the therapeutic nanoparticles can be prolonged by including an agent that delays absorption (e.g., aluminum monostearate and gelatin). Alternatively, controlled release can be achieved by implants and microencapsulated delivery systems, which can include biodegradable, biocompatible polymers (e.g., ethylene vinyl acetate, polyanhydrides, polyglycolic acid, collagen, polyorthoesters, and polylactic acid; Alza Corporation and Nova Pharmaceutical, Inc.).

Compositions containing one or more of any of the therapeutic nanoparticles described herein can be formulated for parenteral (e.g., intravenous, intraarterial, intramuscular, intradermal, subcutaneous, or intraperitoneal) administration in dosage unit form (i.e., physically discrete units containing a predetermined quantity of active compound for ease of administration and uniformity of dosage).

Toxicity and therapeutic efficacy of compositions can be determined by standard pharmaceutical procedures in cell cultures or experimental animals (e.g., monkeys). One can, for example, determine the LD50 (the dose lethal to 50% of the population) and the ED50 (the dose therapeutically effective in 50% of the population): the therapeutic index being the ratio of LD50:ED50. Agents that exhibit high therapeutic indices are preferred. Where an agent exhibits an undesirable side effect, care should be taken to minimize potential damage (i.e., reduce unwanted side effects). Toxicity and therapeutic efficacy can be determined by other standard pharmaceutical procedures.

Data obtained from cell culture assays and animal studies can be used in formulating an appropriate dosage of any given agent for use in a subject (e.g., a human). A therapeutically effective amount of the one or more (e.g., one, two, three, or four) therapeutic nanoparticles (e.g., any of the therapeutic nanoparticles described herein) will be an amount that treats decreases cancer cell invasion or metastasis in a subject having cancer (e.g., breast cancer) in a subject (e.g., a human), treats a metastatic cancer in a lymph node in a subject, decreases or stabilizes metastatic tumor size in a lymph node in a subject, decreases the rate of metastatic tumor growth in a lymph node in a subject, decreases the severity, frequency, and/or duration of one or more symptoms of a metastatic cancer in a lymph node in a subject in a subject (e.g., a human), or decreases the number of symptoms of a metastatic cancer in a lymph node in a subject (e.g., as compared to a control subject having the same disease but not receiving treatment or a different treatment, or the same subject prior to treatment).

The effectiveness and dosing of any of the therapeutic nanoparticles described herein can be determined by a health care professional using methods known in the art, as well as by the observation of one or more symptoms of a metastatic cancer in a lymph node in a subject (e.g., a human). Certain factors may influence the dosage and timing required to effectively treat a subject (e.g., the severity of the disease or disorder, previous treatments, the general health and/or age of the subject, and the presence of other diseases).

Exemplary doses include milligram or microgram amounts of any of the therapeutic nanoparticles described herein per kilogram of the subject's weight. While these doses cover a broad range, one of ordinary skill in the art will understand that therapeutic agents, including the therapeutic nanoparticles described herein, vary in their potency, and effective amounts can be determined by methods known in the art. Typically, relatively low doses are administered at first, and the attending health care professional (in the case of therapeutic application) or a researcher (when still working at the development stage) can subsequently and gradually increase the dose until an appropriate response is obtained. In addition, it is understood that the specific dose level for any particular subject will depend upon a variety of factors including the activity of the specific compound employed, the age, body weight, general health, gender, and diet of the subject, the time of administration, the route of administration, the rate of excretion, and the half-life of the therapeutic nanoparticles *in vivo.*

The pharmaceutical compositions can be included in a container, pack, or dispenser together with instructions for administration.

### Methods of Treatment

The therapeutic nanoparticles described herein were discovered to decrease cancer growth. In view of this discovery, provided herein are said therapeutic nanoparticles and a chemotherapeutic agent for use in treating a cancer in a subject. Specific embodiments and various aspects of these methods are described below.

### Medical use of Heating Cancer

The medical use generally includes identifying a subject who has a tumor, e.g., a cancer. As used herein, the term "cancer" refers to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. In general, a cancer will be associated with the presence of one or more tumors, i.e., abnormal cell masses. The term "tumor" is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. While the present study focused on pancreatic cancer because of its dismal prognosis and the lack of progress against its metastatic form, the present compositions and methods are broadly applicable to solid malignancies. Thus the cancer can be of any type of solid tumor, including but not limited to: breast, colon, kidney, lung, skin, ovarian, pancreatic, rectal, stomach, thyroid, or uterine cancer.

Tumors include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. In some embodiments, the disease is renal carcinoma or melanoma. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

In some embodiments, cancers evaluated or treated by the methods described herein include epithelial cancers, such as a lung cancer (e.g., non-small-cell lung cancer (NSCLC)), breast cancer, colorectal cancer, kidney cancer, head and neck cancer, prostate cancer, pancreatic cancer (e.g., Pancreatic ductal adenocarcinoma (PDAC)) or ovarian cancer. Epithelial malignancies are cancers that affect epithelial tissues.

A cancer can be diagnosed in a subject by a health care professional (e.g., a physician, a physician's assistant, a nurse, or a laboratory technician) using methods known in the art. For example, a metastatic cancer can be diagnosed in a subject, in part, by the observation or detection of at least one symptom of a cancer in a subject as known in the art. A cancer can also be diagnosed in a subject using a variety of imaging techniques (e.g., alone or in combination with the observance of one or more symptoms of a cancer in a subject). For example, the presence of a cancer can be detected in a subject using computer tomography, magnetic resonance imaging, positron emission tomography, and X-ray. A cancer can also be diagnosed by performing a biopsy of tissue from the subject. A cancer can also be diagnosed from serum biomarkers, such as CA19.9, CEA, PSA, etc.

In some embodiments, the methods can include determining whether the cancer expresses or overexpresses an immune checkpoint molecule, e.g., PD-L1. Methods for detecting expression of an immune checkpoint molecule, e.g., PD-L1 in a cancer, e.g., in a biopsy or other sample comprising cells from the cancer, are known in the art, e.g., including commercially available or laboratory-developed immunohistochemistry (IHC); see, e.g., Udall et al., Diagn Pathol. 2018; 13: 12. The level can be compared to a threshold or reference level, and if a level of expression of an immune checkpoint molecule, e.g., PD-L1 above the threshold or reference level are seen, the subject can be selected for a treatment as descried herein. In some embodiments, the methods can include determining whether the cancer has high levels of microsatellite instability (MSI), e.g., as described in Kawakami et al., Curr Treat Options Oncol. 2015 Jul;16(7):30; Zeinalian et al., Adv Biomed Res. 2018; 7: 28, and selecting for treatment a cancer that is MSI-high or that has levels of MSI above a threshold or reference level..

Any one or more of the therapeutic nanoparticles described herein can be administered to a subject having cancer. The one or more therapeutic nanoparticles can be administered to a subject in a health care facility (e.g., in a hospital or a clinic) or in an assisted care facility. In some embodiments, the subject has been previously diagnosed as having a cancer. In some embodiments, the subject has already received therapeutic treatment for the cancer. In some embodiments, one or more tumors has been surgically removed prior to treatment with one of the therapeutic nanoparticles described herein.

In some embodiments, the administering of at least one therapeutic nanoparticle results in a decrease (e.g., a significant or observable decrease) in the size of a tumor, a stabilization of the size (e.g., no significant or observable change in size) of a tumor, or a decrease (e.g., a detectable or observable decrease) in the rate of the growth of a tumor present in a subject. A health care professional can monitor the size and/or changes in the size of a tumor in a subject using a variety of different imaging techniques, including but not limited to: computer tomography, magnetic resonance imaging, positron emission tomography, and X-ray. For example, the size of a tumor of a subject can be determined before and after therapy in order to determine whether there has been a decrease or stabilization in the size of the tumor in the subject in response to therapy. The rate of growth of a tumor can be compared to the rate of growth of a tumor in another subject or population of subjects not receiving treatment or receiving a different treatment. A decrease in the rate of growth of a tumor can also be determined by comparing the rate of growth of a tumor both prior to and following a therapeutic treatment (e.g., treatment with any of the therapeutic nanoparticles described herein). In some embodiments, the visualization of a tumor can be performed using imaging techniques that utilize a labeled probe or molecule that binds specifically to the cancer cells in the tumor (e.g., a labeled antibody that selectively binds to an epitope present on the surface of the cancer cell).

In some embodiments, administering a therapeutic nanoparticle to the subject decreases the risk of developing a metastatic cancer (e.g., a metastatic cancer in a lymph node) in a subject having (e.g., diagnosed as having) a primary cancer (e.g., a primary breast cancer) (e.g., as compared to the rate of developing a metastatic cancer in a subject having a similar primary cancer but not receiving treatment or receiving an alternative treatment). A decrease in the risk of developing a metastatic tumor in a subject having a primary cancer can also be compared to the rate of metastatic cancer formation in a population of subjects receiving no therapy or an alternative form of cancer therapy.

A health care professional can also assess the effectiveness of therapeutic treatment of a cancer by observing a decrease in the number of symptoms of cancer in the subject or by observing a decrease in the severity, frequency, and/or duration of one or more symptoms of a cancer in a subject. A variety of symptoms of a cancer are known in the art and are described herein.

In some embodiments, the administering can result in an increase (e.g., a significant increase) in lifespan or chance of survival or of a cancer in a subject (e.g., as compared to a population of subjects having a similar cancer but receiving a different therapeutic treatment or no therapeutic treatment). In some embodiments, the administering can result in an improved prognosis for a subject having a cancer (e.g., as compared to a population of subjects having a similar cancer r but receiving a different therapeutic treatment or no therapeutic treatment).

### Dosing, Administration, and Compositions

In any of the methods described herein, the therapeutic nanoparticle can be administered by a health care professional (e.g., a physician, a physician's assistant, a nurse, or a laboratory or clinic worker), the subject (i.e., self-administration), or a friend or family member of the subject. The administering can be performed in a clinical setting (e.g., at a clinic or a hospital), in an assisted living facility, or at a pharmacy.

In some embodiments of any of the methods described herein, the therapeutic nanoparticle is administered to a subject that has been diagnosed as having a cancer. In some embodiments, the subject has been diagnosed with breast cancer or pancreatic cancer. In some non-limiting embodiments, the subject is a man or a woman, an adult, an adolescent, or a child. The subject can have experienced one or more symptoms of a cancer or metastatic cancer (e.g., a metastatic cancer in a lymph node). The subject can also be diagnosed as having a severe or an advanced stage of cancer (e.g., a primary or metastatic cancer). In some embodiments, the subject may have been identified as having a metastatic tumor present in at least one lymph node. In some embodiments, the subject may have already undergone surgical resection, e.g., partial or total pancreatectomy, lymphectomy and/or mastectomy.

In some embodiments of any of the methods described herein, the subject is administered at least one (e.g., at least 2, 3, 4, 5, 6, 7, 8, 9, 10, 15, 20, 25, or 30) dose of a composition containing at least one (e.g., one, two, three, or four) of any of the magnetic particles or pharmaceutical compositions described herein. In any of the methods described herein, the at least one magnetic particle or pharmaceutical composition (e.g., any of the magnetic particles or pharmaceutical compositions described herein) can be administered intravenously, intaarterially, subcutaneously, intraperitoneally, or intramuscularly to the subject. In some embodiments, the at least magnetic particle or pharmaceutical composition is directly administered (injected) into a lymph node in a subject.

In some embodiments, the subject is administered at least one therapeutic nanoparticle or pharmaceutical composition (e.g., any of the therapeutic nanoparticles or pharmaceutical compositions described herein) and at least one additional therapeutic agent. The at least one additional therapeutic agent can be a chemotherapeutic agent. By the term "chemotherapeutic agent" is meant a molecule that can be used to reduce the rate of cancer cell growth or to induce or mediate the death (e.g., necrosis or apoptosis) of cancer cells in a subject (e.g., a human). In non-limiting examples, a chemotherapeutic agent can be a small molecule, a protein (e.g., an antibody, an antigen-binding fragment of an antibody, or a derivative or conjugate thereof), a nucleic acid, or any combination thereof. Non-limiting examples of chemotherapeutic agents include one or more alkylating agents; anthracyclines; cytoskeletal disruptors (taxanes); epothilones; histone deacetylase inhibitors; inhibitors of topoisomerase I; inhibitors of topoisomerase II; kinase inhibitors; nucleotide analogs and precursor analogs; peptide antibiotics; platinum-based agents; retinoids; and/or vinca alkaloids and derivatives; or any combination thereof. In some embodiments, the chemotherapeutic agent is a nucleotide analog or precursor analog, e.g., azacitidine; azathioprine; capecitabine; cytarabine; doxifluridine; fluorouracil; gemcitabine; hydroxyurea; mercaptopurine; methotrexate; or tioguanine. Other examples include cyclophosphamide, mechlorethamine, chlorabucil, melphalan, daunorubicin, doxorubicin, epirubicin, idarubicin, mitoxantrone, valrubicin, paclitaxel, docetaxel, etoposide, teniposide, tafluposide, bleomycin, carboplatin, cisplatin, oxaliplatin, all-trans retinoic acid, vinblastine, vincristine, vindesine, vinorelbine, and bevacizumab (or an antigen-binding fragment thereof). Additional examples of chemotherapeutic agents are known in the art.

In some embodiments, the chemotherapeutic agent is chosen based on the cancer type or based on genetic analysis of the cancer; for example, for pancreatic cancer, one or more of ABRAXANE (albumin-bound paclitaxel), Gemzar (gemcitabine), capecitabine, 5-FU (fluorouracil) and ONIVYDE (irinotecan liposome injection), or combinations thereof, e.g., FOLFIRINOX, a combination of three chemotherapy drugs (5-FU/leucovorin, irinotecan and oxaliplatin), or modified FOLFIRINOX (mFOLFIRINOX) can be administered. Further combinations of targets that may work synergistically by complementary mechanisms could be used. For example, combination therapies can be used that physically alter the tumor microenviroment by enzymatic degradation via recombinant human hyaluronidase (PEGPH20),^{30,31} or other alternative chemotherapy agents, and/or alternative checkpoint inhibitors that may promote a synergistic effect in activating T-cells (e.g., anti-PD-1 and/or anti-CTLA-4).

The methods and compositions can also include administration of an analgesic (e.g., acetaminophen, diclofenac, diflunisal, etodolac, fenoprofen, flurbiprofen, ibuprofen, indomethacin, ketoprofen, ketorolac, meclofenamate, mefenamic acid, meloxicam, nabumetone, naproxen, oxaprozin, phenylbutazone, piroxicam, sulindac, tolmetin, celecoxib, buprenorphine, butorphanol, codeine, hydrocodone, hydromorphone, levorphanol, meperidine, methadone, morphine, nalbuphine, oxycodone, oxymorphone, pentazocine, propoxyphene, and tramadol).

In some embodiments, at least one additional therapeutic agent and at least one therapeutic nanoparticle (e.g., any of the therapeutic nanoparticles described herein) are administered in the same composition (e.g., the same pharmaceutical composition). In some embodiments, the at least one additional therapeutic agent and the at least one therapeutic nanoparticle are administered to the subject using different routes of administration (e.g., at least one additional therapeutic agent delivered by oral administration and at least one therapeutic nanoparticle delivered by intravenous administration).

In any of the methods described herein, the at least one therapeutic nanoparticle or pharmaceutical composition (e.g., any of the therapeutic nanoparticles or pharmaceutical compositions described herein) and, optionally, at least one additional therapeutic agent can be administered to the subject at least once a week (e.g., once a week, twice a week, three times a week, four times a week, once a day, twice a day, or three times a day). In some embodiments, at least two different therapeutic nanoparticles are administered in the same composition (e.g., a liquid composition). In some embodiments, at least one therapeutic nanoparticle and at least one additional therapeutic agent are administered in the same composition (e.g., a liquid composition). In some embodiments, the at least one therapeutic nanoparticle and the at least one additional therapeutic agent are administered in two different compositions (e.g., a liquid composition containing at least one therapeutic nanoparticle and a solid oral composition containing at least one additional therapeutic agent). In some embodiments, the at least one additional therapeutic agent is administered as a pill, tablet, or capsule. In some embodiments, the at least one additional therapeutic agent is administered in a sustained-release oral formulation.

In some embodiments, the one or more additional therapeutic agents can be administered to the subject prior to administering the at least one therapeutic nanoparticle or pharmaceutical composition (e.g., any of the therapeutic nanoparticles or pharmaceutical compositions described herein). In some embodiments, the one or more additional therapeutic agents can be administered to the subject after administering the at least one therapeutic nanoparticle or pharmaceutical composition (e.g., any of the magnetic particles or pharmaceutical compositions described herein). In some embodiments, the one or more additional therapeutic agents and the at least one therapeutic nanoparticle or pharmaceutical composition (e.g., any of the therapeutic nanoparticles or pharmaceutical compositions described herein) are administered to the subject such that there is an overlap in the bioactive period of the one or more additional therapeutic agents and the at least one therapeutic nanoparticle (e.g., any of the therapeutic nanoparticles described herein) in the subject.

In some embodiments, the subject can be administered the at least one therapeutic nanoparticle or pharmaceutical composition (e.g., any of the therapeutic nanoparticles or pharmaceutical compositions described herein) over an extended period of time (e.g., over a period of at least 1 week, 2 weeks, 3 weeks, 1 month, 2 months, 3 months, 4 months, 5 months, 6 months, 7 months, 8 months, 9 months, 10 months, 11 months, 12 months, 1 year, 2 years, 3 years, 4 years, 5 years, or 10 years). A skilled medical professional may determine the length of the treatment period using any of the methods described herein for diagnosing or following the effectiveness of treatment (e.g., using the methods above and those known in the art). As described herein, a skilled medical professional can also change the identity and number (e.g., increase or decrease) of therapeutic nanoparticles (and/or one or more additional therapeutic agents) administered to the subject and can also adjust (e.g., increase or decrease) the dosage or frequency of administration of at least one therapeutic nanoparticle (and/or one or more additional therapeutic agents) to the subject based on an assessment of the effectiveness of the treatment (e.g., using any of the methods described herein and known in the art). A skilled medical professional can further determine when to discontinue treatment (e.g., for example, when the subject's symptoms are significantly decreased).

### Monitoring Therapy

An additional key advantage of our therapeutic approach derives from the fact that it presents the unique opportunity to develop a clinically-relevant, image-guided treatment protocol that combines knowledge about drug bioavailability in the target tissue and therapeutic outcome. This capability is made possible by the fact that MN-siPDL1 incorporates a 20-nm superparamagnetic nanoparticle carrier, which ensures highly efficient delivery to tumor cells and whose accumulation in tissues can be monitored by quantitative noninvasive MRI. This capability is unique in the context of all other available therapeutic approaches, which do not present the possibility of noninvasively measuring drug bioavailability during treatment.

As illustrated in the present study, knowledge about relative concentration of MN-siPDL1in tissue through the delta-R2 parameter allowed the development and optimization of a therapeutic protocol associated with durable tumor regression. Concurrently, anatomical MRI allowed the objective measurement of tumor volume as a morphologic biomarker of response. However, the application of dynamic MR imaging protocols could readily be used to also measure physiologic variables related to tumor blood flow and microvessel permeability.

Thus the present methods can include the use of imaging modalities that detect the magnetic nanoparticles.

### EXAMPLES

The invention is further described in the following examples, which do not limit the scope of the invention described in the claims.

### METHODS

### Small interfering RNA (siRNA) oligos

The sequence of the siRNA oligo against *pd-l1* (siPDL1; MW = 13,788.9 g/mol), consisted of 5'-ThioMC6-D/GGUCAACGCCACAGCGAAUUU-3' (sense sequence; SEQ ID NO:2) and 5'-PAUUCGCUGUGGCGUUGACCUU-3' (anti-sense sequence; SEQ ID NO:3). The sequence of the scrambled siRNA oligo (siSCR; MW = 13,728.8 g/mol) was 5'-ThioMC6-D/UGGUUUACAUGUCGACUAAUU-3' (sense sequence; SEQ ID NO:4) and 5'-PUUAGUCGACAUGUAAACCAUU-3' (anti-sense sequence; SEQ ID NO:5). Both siRNAs were designed and synthesized by Dharmacon (Lafayette, CO). The 5'-Thiol-Modifier C6 disulfide (5'-ThioMC6) was introduced in sense sequences for conjugating to magnetic nanoparticles.

### Synthesis of dextran coated magnetic nanoparticles (MN)

MN was synthesized following a protocol published previously ²⁰. Briefly, 30ml of Dextan-T10 (0.3 g ml⁻¹, Pharmacosmos A/S, Holbaek, Denmark) was mixed with 1ml of FeCl₃.6H₂O (0.65 g ml⁻¹, Sigma, Saint Louis, MO) while flushing Argon gas for an hour. 1ml of FeCl₂.4H₂O (0.4 g ml⁻¹, Sigma) was added into the mixture and 15ml of cold NH₄OH (28%, Sigma) was added dropwise to the stirred mixture. The temperature was increased to 85 °C for 1 h to start the formation of a nanoparticulate dispersion and then cooled to room temperature. The magnetic nanoparticles were concentrated to 20 ml using Amicon ultra centrifugal units (MWCO 30 kDa; Millipore, Darmstadt, Germany). The resulting dextran-coated magnetic nanoparticles were cross-linked by epichlorohydrin (14 ml, 8 h, Sigma) and aminated with subsequent addition of NH₄OH (28%, 60 ml). Aminated magnetic nanoparticles (MN) were purified by dialysis and concentrated using Amicon ultra centrifugal units. The properties of MN were as follows: concentration, 10.94 mg ml⁻¹ as Fe; the number of amine groups per MN, 64; relaxivity (R2), 82.5 mM⁻¹sec⁻¹; size of MN, 20.3±0.6 nm (NanoSight LM-10 system and Nanoparticles Tracking Analysis software (Ver. 3.2), Malvern, UK).

### Nanodrug Synthesis and Characterization

Nanodrug synthesis was carried out according to a previously published protocol ²⁰. See also Fig. 1A and Fig. 6. Briefly, MN was conjugated to the heterobifunctional linker N-Succinimidyl 3-[2-pyridyldithio]-propionate (SPDP, Thermoscientific Co., Rockford, IL), which was utilized for the linkage of activated siRNA oligos. SPDP was dissolved in anhydrous DMSO and incubated with MN, which has a pyridyldithio group to form a reduction labile disulfide linkage with the siRNA oligos. The 5'-ThioMC6 of the siRNA oligo was activated to release the thiol via 3% TCEP (Thermoscientific Co., Rockford, IL) treatment in nuclease free PBS. The siRNA oligos were purified using an ammonium acetate/ethanol precipitation method. After TCEP-activation and purification, each oligo (siPDL1 and siSCR) was dissolved in water and incubated with the SPDP modified MN overnight to prepare nanodrugs (MN-siPDL1 and MN-siSCR). Oligos were added to MN at two different ratios to obtain nanodrugs incorporating 2.1±0.4 (low-dose) or 4.8±0.7 (high-dose) siRNA oligos per MN, as quantified by the electrophoresis analysis method ²⁰. Nanodrug was freshly prepared each week. The size of the final MN-siPDL1/SCR was 23.2±0.9 nm.

### Cell lines

The murine pancreatic ductal adenocarcinoma, PAN 02 cell line (NCI, Frederick, MD) was cultured in RPMI 1640 culture medium (Sigma), supplemented with 10% FBS (Thermoscientific, Waltham, MA), 1% antibiotics (Invitrogen, Carlsbad, CA), and 2 mM L-glutamine, per the supplier's instructions. The medium was changed 3 times per week and trypsinized for sub-culturing once per week.

### Immunohistological Tissue staining and Fluorescence Microscopy

Primary and secondary antibodies were purchased from Abcam (Cambridge, MA) and included: anti-CD3 (Cat. #: AB16669), anti-CD8 (Cat. #: AB25478), anti-FoxP3 (Cat. #: AB75763), Granzyme B (Cat. #: AB4069), anti-Ki67 (Cat. #: AB16667), and anti-PDL1 (Cat. #: AB80276) as primary antibodies, Goat Anti-Rat IgG H&L (DyLight 488 pre-adsorbed, AB98420) and Goat Anti-Rabbit IgG H&L (DyLight 488 pre-adsorbed, AB96899) as secondary antibodies.

The immunohistological tissue staining was performed following the protocol for each biomarker. Briefly, excised tumor tissues were embedded in Tissue-Tek OCT compound (Sakura Finetek, Torrance, CA) and snap frozen in liquid nitrogen. The tissues were cut into 7 µm sections and fixed in 4% formaldehyde for 10 min. Detergent permeabilization was performed using 0.1% Triton X-100 in PBS, when needed. After blocking with 5% goat serum in 0.5% bovine serum albumin in PBS, each slide was incubated with corresponding primary antibody (dilution 1/200) at 5°C overnight. Each slide was incubated with secondary antibody (dilution 1/200) for 60 min and mounted with Vectashield mounting medium with DAPI (Vector Laboratories, Inc. Burlingame, CA). The slides were analyzed using a Nikon E400 fluorescence microscope (Nikon, Tokyo, Japan), equipped with the necessary filter sets (MVI Inc., Avon, MA). Images were acquired using a charge coupled device camera with near-IR sensitivity (SPOT 7.4 Slider RTKE; Diagnostic Instruments, Sterling Heights, MI). The images were analyzed using SPOT 4.0 Advance version software (Diagnostic Instruments) and ImageJ (Ver. 1.51c, NIH).

### In vivo MR Imaging

MR imaging was performed before and after each weekly treatment with MN-siPDL1/SCR using a Bruker 9.4T horizontal bore magnet (Magnex Scientific) with gradient insert and operated using ParaVision 5.1 software. Mice formed tumors 2 weeks after inoculation and were monitored by MR imaging for the quantitative measurement of tumor volumes, utilizing a rapid acquisition with relaxation enhancement (RARE) T₁ weighted protocol (TE = 8.5 msec, TR = 2500 msec, number of average = 4, RARE factor = 4, FOV = 4 x 4 cm², matrix size = 128 x 128 pixels, number of slices = 50, slice thickness = 0.5mm, and interslice thickness = 0.5mm) and T₂ weighted protocol (TE = 8.5 msec, TR = 7500 msec, number of average = 4, RARE factor = 16, FOV = 4 x 4 cm², matrix size = 128 x 128 pixels, number of slices = 50, slice thickness = 0.5mm, and interslice thickness = 0.5mm, flip angle = 162 degree). Multi slice multi-echo (MSME) T2-weighted maps were collected with the following parameters: TE=8, 16, 24, 32, 40, 48, 56, 64, 72, and 80 msec, TR=4500 msec, number of slices = 5, slice orient = axial, number of average = 1, RARE factor = 1, field of view = 4 x 4 cm², matrix size = 128 x 128 pixels, slice thickness = 0.5 mm, interslice thickness = 0.5 mm, flip angle = 128 degree). The measurement of tumor volumes and the reconstructions of the T2 maps were performed by two independent investigators blinded to sample identity to account for variability in region of interest (ROI) selection. T2 maps and T2 relaxation times in the tumors were calculated using ImageJ software (Ver. 1.50c, NIH). The relaxation rate R2 was obtained as a reciprocal of relaxation time. Consequently, the change of relaxation rate (delta R2) is proportional to the concentration of iron oxide in MN. Delta R2 was calculated using the following equations: S=S₀ Exp(TE/T₂), ΔR2= 1/T_{2,1}- 1/T_{2,2}=(1/TE)*ln(S₁/S₂), and correlated to the concentration of MN following the equation: ΔR2= r2•Δ[C]. All delta R2 values were calculated relative to week 0.

### Animal model

Six-week-old female mice (C57Bl/6, n = 12) were implanted in the right flank with a murine pancreatic cancer cell line, Pan02 cells (0.25 x 10⁶ cells). One week after cell injection, tumor size was monitored by caliper measurements. Tumor volume was calculated according to the equation: Volume = 0.5 x L x W², where L is length, and W, width. Treatment was initiated once tumor volumes reached 50 mm³, as estimated using calipers. Thereafter, tumor volume was measured by MRI once mice were enrolled in the study and before and after each weekly treatment. All animal experiments were performed in compliance with institutional guidelines and approved by the Institutional Animal Care and Use Committee at Massachusetts General Hospital.

### Therapy

Mice (n = 6) were randomly assigned to an experimental group (treated with MN-siPDL1+ gemcitabine) or a control group (treated with MN-siSCR + gemcitabine). Mice in the experimental group were treated with low-dose MN-siPDL1 (10 mg kg⁻¹ as iron; 520 nmoles/kg siRNA) in solution with gemcitabine (333.3 mg/kg), or high-dose MN-siPDL1(10 mg kg⁻¹ as iron; 937 nmoles/kg siRNA) in solution with gemcitabine (333.3 mg/kg). Mice in the control group received MN-siSCR and gemcitabine at the same doses. In both cases, the drugs were administered as a mixture of nanodrug and gemcitabine through tail vein (i.v. injection) weekly bases. After week 7, co-administration of gemcitabine was discontinued to avoid exceeding the maximum tolerated dose, and only nanodrug was administered until the end of the study. All mice were monitored weekly by magnetic resonance imaging to keep track of changes in tumor volume for a maximum of 12 weeks after the first treatment or until animals became moribund.

### Statistical Analysis.

Data were expressed as mean ± s.d. or s.e.m., where indicated. Statistical comparisons were drawn using a two-tailed t-test (SigmaStat 3.0; Systat Software, Richmond, CA). A value of P < 0.05 was considered statistically significant.

### Example 1. RNAi-Mediated PD-L1 Inhibition for Pancreatic Cancer Immunotherapy.

MN-siPDL1can be delivered in vivo to primary tumors and the delivery can be monitored by noninvasive MRI.

In order to successfully deliver therapeutic amounts of siRNA to tumor cells following intravenous injection, we needed to optimize the design of MN-siPDL1 in terms of hydrodynamic size, conjugation method, and number of siRNA oligos per nanoparticle. An exemplary synthetic scheme of MN-siPDL1 is illustrated in Fig. 1A. To ensure long circulation times (>4 hrs.) and efficient diffusion across the vascular endothelium and throughout the tumor interstitium, we designed MN-siPDL1so that its final size after sequential conjugation to the SPDP linker and the oligo was 23.2±0.9 nm. The number of siRNA oligonucleotides per nanoparticle was adjusted to no more than 4.8±0.7 with the goal of minimizing steric interference with bioconjugation. This design is optimized to enhance the uptake of the nanodrug by tumor tissue through the Enhanced Permeation-Retention (EPR) effect. In addition, the SPDP linker was chosen due to its reducible nature, which ensures dissociation of the oligo from the nanoparticle in cancer cells and efficient entry into the RNA-induced silencing complex (RISC). Finally, to permit detection of MN-siPDL1 by magnetic resonance imaging, the relaxivity (R2) of the final preparation was adjusted by varying the ratios of [Fe³⁺]/[Fe^{2 +}] to achieve an R2 of 82.5 mM⁻¹sec⁻¹ (Fig. 1B).

For the purposes of establishing an effective therapeutic protocol, we needed to confirm delivery of MN-siPDL1 to pancreatic tumor tissue and to demonstrate the capability of MRI to semi-quantitatively measure MN-siPDL1 bioavailability in tumors. We tested our hypothesis using the PAN02 syngeneic pancreatic cancer model. These animals formed tumors 2 weeks after inoculation and were monitored by MR imaging, using single-echo and multi-echo T₂ weighted protocols. The difference in relaxation rate (Delta R₂) was calculated using the following equations: S=S0 Exp(TE/T2), ΔR₂= 1/T2,1- 1/T2,2=(1/TE)*Ln(S1/S2) and ΔR₂= r₂•△[C]. For the visualization of MN-siPDL1 distribution in tumor tissue, T2 maps were reconstructed from the multiecho T₂-weighted image set.

As shown in Fig 2A, the localization of MN-siPDL1 in tumor tissue caused shortening of the T2 relaxation time and resulted in negative contrast as compared to the pre-contrast image. The delta R₂- derived concentration of MN-siPDL1 showed a linear increase during the first three weeks. The accumulation rate of MN-siPDL1 was 1.5-fold faster than that of MN-siSCR during that time period (Fig. 2B). Since the concentration of the nanodrug in tumor cells reflects mostly dilution due to cell division, the faster growth rate of the control tumors treated with MN-siSCR likely led to the observed slower increase in concentration over time in this group. This difference was more pronounced at the later stages of tumor growth, further supporting this hypothesis. The rate of concentration decrease, reflective of rapid nanodrug dilution due to tumor cell division in the control group treated with MN-siSCR, was 5.1-fold greater than in the experimental group treated with MN-siPDL1, indicating a more rapid growth of the tumor in the control animals (Fig. 2C).

Combination treatment with gemcitabine and MN-siPDL1 is effective in a model of syngeneic pancreatic cancer

Our therapeutic studies illustrated the potential of the combination treatment with gemcitabine and MN-siPDL1in pancreatic cancer. In these studies, a syngeneic model of pancreatic cancer was generated by implanting the murine pancreatic cancer cell line PAN02 in the right flank of six-week old, female C57BL/6 mice.

To determine whether treatment with gemcitabine in combination of MN-siPDL1 could inhibit tumor growth, the mice were treated with gemcitabine in solution with a low dose of MN-siPDL1 or siSCR (10mg/kg Fe; 520nmoles/kg siRNA in both groups) or a high dose of MN-siPDL1 or siSCR (10 mg/kg Fe, 937nmoles/kg siRNA in both groups) delivered intravenously through the tail vein (i.v.). The combination treatment was initiated when the tumor size reached >50mm³ as measured by anatomical MR imaging and continued for 12 weeks. In all of the therapeutic studies, the change in tumor volume was monitored by anatomical MR imaging before the administration of each weekly treatment (Fig 3A).

The mice co-treated with MN-siPDL1and gemcitabine demonstrated significant inhibition of tumor growth, relative to the inactive MN-siSCR controls (P < 0.05). This difference was evident at week 2 from the beginning of treatment, when tumor volume had decreased from 52.8±6.7 mm³ in week 0 to 5.3±0.8 mm³ in week 2 (p = 0.012). The difference persisted for the duration of the study (p <0.05). Tumor volumes in the low-dose group were not different from the MN-siSCR control until week 6 (Figs. 3A-B and Figs. 5A-D).

In the high-dose MN-siPDL1 group, 67% of the mice showed objective response to treatment defined as inhibition of tumor growth. 33% of the mice failed to respond and died after 6 weeks, indicating variability of the response. The time constants of tumor growth stratifying the experimental animals according to response are presented in Table I.

**Table I. Tumor Growth Rate Constants in Animals treated with MN-siPDL1or MN-siSCR and Gemcitabine.**

| **Treatment Group** | **Tumor growth rate constant (week-¹)** |
|---|---|
| *High-dose MN-siPDL1*+*gemcitabine (responder)* | 0.0813 |
| *High-dose MN-siPDL1+gemcitabine (non-responder)* | 0.5427 |
| *Low-dose MN-siPDL1+gemcitabine* | 0.3627 |
| *MN-siSCR+gemcitabine* | 0.5562 |

Finally, the advantage of the combination treatment was clearly seen when assessing animal survival (Fig 3C). 67% of the mice treated with gemcitabine and MN-siPDL1(high dose) survived until week 12. 67% of the mice treated with gemcitabine and MN-siPDL1(low dose) survived until week 8. All of the control mice treated with MN-siSCR and gemcitabine succumbed by week 6.

Interestingly, all of the mice in the group treated with gemcitabine and MN-siSCR developed large necrotic tumors, presumably due to the high rate of tumor growth. Tumor necrosis and ulceration was not seen in the experimental animals (Fig. 3D).

### Combination treatment prevented the inactivation of cytotoxic T cells

In order to assess the effect of treatment on the anti-tumor immune response, we analyzed tissue biomarkers of immune cell recruitment and activation in the tumors of treated mice. After combination treatment with MN-siPDL1and gemcitabine, PD-L1 expression was significantly reduced. There was evidence of recruitment of CD8+ tumor infiltrating lymphocytes (TILs) and an increase in cell-mediated cytotoxicity, as evidenced by higher levels of Granzyme B. The tumor infiltration by immunosuppressive Foxp3+ regulatory T (Treg) cells was also significantly reduced. Finally, tumor cell proliferation was inhibited (Figs. 4A-B). Interestingly, the expression of these biomarkers in non-responsive animals treated with high-dose MN-siPDL1and gemcitabine, was intermediate between the control animals and the regressing experimental animals, suggesting that there is a critical level of PD-L1 inhibition needed in order to observe macroscopic response (Figs. 4A-B). These results suggested that the observed therapeutic effect was the result of successful induction of an anti-tumor immune response.

### REFERENCES

1 Sheahan, A. V., Biankin, A. V., Parish, C. R. & Khachigian, L. M. Targeted therapies in the management of locally advanced and metastatic pancreatic cancer: a systematic review. Oncotarget 9, 21613-21627 (2018).
2 Oberstein, P. E. & Olive, K. P. Pancreatic cancer: why is it so hard to treat? Therap Adv Gastroenterol 6, 321-337 (2013).
3 McAllister, F. et al. Current Status and Future Directions for Screening Patients at High Risk for Pancreatic Cancer. Gastroenterol Hepatol (N Y) 13, 268-275 (2017).
4 Dauer, P., Nomura, A., Saluja, A. & Banerjee, S. Microenvironment in determining chemo-resistance in pancreatic cancer: Neighborhood matters. Pancreatology 17, 7-12 (2017).
5 Erstad, D. J. et al. Orthotopic and heterotopic murine models of pancreatic cancer and their different responses to FOLFIRINOX chemotherapy. Dis Model Mech 11 (2018).
6 Conroy, T. et al. FOLFIRINOX versus gemcitabine for metastatic pancreatic cancer. N Engl J Med 364, 1817-1825 (2011).
7 Vogel, A. et al. Efficacy and safety profile of nab-paclitaxel plus gemcitabine in patients with metastatic pancreatic cancer treated to disease progression: a subanalysis from a phase 3 trial (MPACT). BMC Cancer 16, 817 (2016).
8 Wilden, S. M., Lang, B. M., Mohr, P. & Grabbe, S. Immune checkpoint inhibitors: a milestone in the treatment of melanoma. J Dtsch Dermatol Ges 14, 685-695 (2016).
9 Pabani, A. & Butts, C. A. Current landscape of immunotherapy for the treatment of metastatic non-small-cell lung cancer. Curr Oncol 25, S94-S102 (2018).
10 Beatty, G. L. & Gladney, W. L. Immune escape mechanisms as a guide for cancer immunotherapy. Clin Cancer Res 21, 687-692 (2015).
11 Ghosh, S. K. et al. Targeted imaging of breast tumor progression and therapeutic response in a human uMUC-1 expressing transgenic mouse model. Int J Cancer 132, 1860-1867 (2013).
12 Ghosh, S. K. et al. Sequence-dependent combination therapy with doxorubicin and a survivin-specific small interfering RNA nanodrug demonstrates efficacy in models of adenocarcinoma. Int J Cancer 134, 1758-1766 (2014).
13 Kumar, M., Yigit, M., Dai, G., Moore, A. & Medarova, Z. Image-guided breast tumor therapy using a small interfering RNA nanodrug. Cancer Res 70, 7553-7561 (2010).
14 Medarova, Z., Pham, W., Farrar, C., Petkova, V. & Moore, A. In vivo imaging of siRNA delivery and silencing in tumors. Nat Med 13, 372-377 (2007).
15 Yigit, M. V. et al. Context-dependent differences in miR-10b breast oncogenesis can be targeted for the prevention and arrest of lymph node metastasis. Oncogene 32, 1530-1538 (2013).
16 Yigit, M. V., Moore, A. & Medarova, Z. Magnetic nanoparticles for cancer diagnosis and therapy. Pharm Res 29, 1180-1188 (2012).
17 Yoo, B. et al. Design of nanodrugs for miRNA targeting in tumor cells. J Biomed Nanotechnol 10, 1114-1122 (2014).
18 Yoo, B. et al. Combining miR-10b-Targeted Nanotherapy with Low-Dose Doxorubicin Elicits Durable Regressions of Metastatic Breast Cancer. Cancer Res 75, 4407-4415 (2015).
19 Yoo, B. et al. Therapy targeted to the metastatic niche is effective in a model of stage IV breast cancer. Sci Rep 7, 45060 (2017).
20 Medarova, Z., Balcioglu, M. & Yigit, M. V. Controlling RNA Expression in Cancer Using Iron Oxide Nanoparticles Detectable by MRI and In Vivo Optical Imaging. Methods Mol Biol 1372, 163-179 (2016).
21 Yoo, B., Ifediba, M. A., Ghosh, S., Medarova, Z. & Moore, A. Combination treatment with theranostic nanoparticles for glioblastoma sensitization to TMZ. Mol Imaging Biol 16, 680-689 (2014).
22 Powles, T. et al. Efficacy and Safety of Durvalumab in Locally Advanced or Metastatic Urothelial Carcinoma: Updated Results From a Phase 1/2 Open-label Study. JAMA Oncol 3, e172411 (2017).
23 Crist, M. & Balar, A. Atezolizumab in invasive and metastatic urothelial carcinoma. Expert Rev Clin Pharmacol 10, 1295-1301 (2017).
24 Balar, A. V. et al. Atezolizumab as first-line treatment in cisplatin-ineligible patients with locally advanced and metastatic urothelial carcinoma: a single-arm, multicentre, phase 2 trial. Lancet 389, 67-76 (2017).
25 Powles, T. et al. Atezolizumab versus chemotherapy in patients with platinum-treated locally advanced or metastatic urothelial carcinoma (IMvigor211): a multi centre, open-label, phase 3 randomised controlled trial. Lancet 391, 748-757 (2018).
26 AstraZeneca. AstraZeneca reports initial results from the ongoing MYSTIC trial in Stage IV lung cancer. . (2016).
27 Royal, R. E. et al. Phase 2 trial of single agent Ipilimumab (anti-CTLA-4) for locally advanced or metastatic pancreatic adenocarcinoma. J Immunother 33, 828-833 (2010).
28 Brahmer, J. R. et al. Safety and activity of anti-PD-L1 antibody in patients with advanced cancer. N Engl J Med 366, 2455-2465 (2012).
29 Renouf, D. J. D., N.C.; Kavan, P.; Jonker, D.J.; Chia-chi, W. A.; Hsu, T. The Canadian Cancer Trials Group PA.7 trial: results from the safety run in of a randomized phase II study of gemcitabine (GEM) and nab-paclitaxel (Nab-P) versus GEM, nab-P, durvalumab (D), and tremelimumab (T) as first-line therapy in metastatic pancreatic ductal adenocarcinoma (mPDAC). J Clin Oncol 36 (2018).
30 Doherty, G. J., Tempero, M. & Corrie, P. G. HALO-109-301: a Phase III trial of PEGPH20 (with gemcitabine and nab-paclitaxel) in hyaluronic acid-high stage IV pancreatic cancer. Future Oncol 14, 13-22 (2018).
31 Neesse, A., Algul, H., Tuveson, D. A. & Gress, T. M. Stromal biology and therapy in pancreatic cancer: a changing paradigm. Gut 64, 1476-1484 (2015).

## Claims

1. A therapeutic nanoparticle, wherein said nanoparticle:
has a diameter of between 10 nm to 30 nm; and
comprises an iron oxide core, a polymer coating comprising dextran, and an inhibitory nucleic acid targeting an immune checkpoint molecule, wherein the immune checkpoint molecule is programmed cell death 1 ligand 1 (PD-L1), that is covalently linked to the nanoparticle.

2. The therapeutic nanoparticle of claim 1, wherein the nucleic acid comprises a sequence of at least 10 contiguous nucleotides complementary to SEQ ID NO:1.

3. The therapeutic nanoparticle of claim 1, wherein the nucleic acid comprises at least one modified nucleotide, wherein the at least one modified nucleotide comprises at least one modification in its base and/or at least one modification in its sugar, or wherein the at least one modified nucleotide comprises a modification in an atom that forms a phosphodiester bond between two adjoining nucleotides in the nucleic acid.

4. The therapeutic nanoparticle of claim 3, wherein the at least one modified nucleotide is a locked nucleotide.

5. The therapeutic nanoparticle of claim 1, wherein the nucleic acid is a small interfering RNA (siRNA) molecule.

6. The therapeutic nanoparticle of claim 1, wherein the nucleic acid is covalently-linked to the nanoparticle through a chemical moiety comprising a disulfide bond or a thioether bond.

7. The therapeutic nanoparticle of claim 1, wherein the nanoparticle is magnetic.

8. A pharmaceutical composition comprising the therapeutic nanoparticle of any of claims 1-7, and optionally a chemotherapeutic agent, optionally wherein the chemotherapeutic agent is gemcitabine.

9. The therapeutic nanoparticle of claims 1-7, and a chemotherapeutic agent, for use in treating cancer in a subject.

10. The therapeutic nanoparticle and the chemotherapeutic agent for use of claim 9, wherein the cancer is selected from the group consisting of: breast cancer, colon cancer, kidney cancer, lung cancer, skin cancer, ovarian cancer, pancreatic cancer, prostate cancer, rectal cancer, stomach cancer, thyroid cancer, and uterine cancer.

11. The therapeutic nanoparticle and the chemotherapeutic agent for use of claim 9, further comprising imaging a tissue of the subject to determine a location or number of cancer cells in the subject, or a location of the therapeutic nanoparticles in the subject.

12. The therapeutic nanoparticle and the chemotherapeutic agent for use of claim 9, wherein the therapeutic nanoparticle is administered in two or more doses to the subject, optionally wherein the therapeutic nanoparticle is administered to the subject at least once a week.

13. The therapeutic nanoparticle and the chemotherapeutic agent for use of claim 9, wherein the therapeutic nanoparticle is administered to the subject by intravenous, subcutaneous, intraarterial, intramuscular, or intraperitoneal administration.

14. The therapeutic nanoparticle and the chemotherapeutic agent for use of claim 9, wherein the subject has pancreatic cancer.

15. The therapeutic nanoparticle and the chemotherapeutic agent for use of claim 9, wherein the chemotherapeutic agent is gemcitabine.

## Patentansprüche

1. Therapeutischer Nanopartikel, wobei der Nanopartikel:
einen Durchmesser hat, der zwischen 10 nm bis 30 nm beträgt; und
einen Kern aus Eisenoxid, eine Polymerbeschichtung, welche Dextran umfasst, und eine hemmend wirkende Nukleinsäure umfasst, welche gegen ein Immuncheckpoint-Molekül gerichtet ist, wobei es sich bei dem Immuncheckpoint-Molekül um den programmierten Zelltod-1-Liganden 1 (PD-L1) handelt, wobei sie kovalent an den Nanopartikel gebunden ist.

2. Therapeutischer Nanopartikel nach Anspruch 1, wobei die Nukleinsäure eine Sequenz aus mindestens 10 zusammenhängenden Nukleotiden umfasst, die komplementär zur SEQ ID Nr. 1 ist.

3. Therapeutischer Nanopartikel nach Anspruch 1, wobei die Nukleinsäure mindestens ein modifiziertes Nukleotid umfasst, wobei das mindestens eine modifizierte Nukleotid mindestens eine Modifizierung an seiner Base und/oder mindestens eine Modifizierung an seinem Zucker umfasst, oder wobei das mindestens eine modifizierte Nukleotid eine Modifizierung an einem Atom umfasst, welches eine Phosphodiester-Bindung zwischen zwei benachbarten Nukleotiden in der Nukleinsäure bildet.

4. Therapeutischer Nanopartikel nach Anspruch 3, wobei es sich bei dem mindestens einen modifizierten Nukleotid um ein verbrücktes Nukleotid handelt.

5. Therapeutischer Nanopartikel nach Anspruch 1, wobei es sich bei der Nukleinsäure um Molekül des Typs Small interfering RNA (siRNA) handelt.

6. Therapeutischer Nanopartikel nach Anspruch 1, wobei die Nukleinsäure über einen chemischen Baustein, welcher eine Disulfidbindung oder eine Thioetherbindung umfasst, kovalent an den Nanopartikel gebunden ist.

7. Therapeutischer Nanopartikel nach Anspruch 1, wobei der Nanopartikel magnetisch ist.

8. Pharmazeutische Zusammensetzung, die den therapeutischen Nanopartikel nach beliebigen der Ansprüche 1 bis 7 und wahlweise ein chemotherapeutisches Mittel umfasst, wobei es sich bei dem chemotherapeutischen Mittel um Gemcitabin handelt.

9. Therapeutischer Nanopartikel nach den Ansprüchen 1 bis 7 sowie ein chemotherapeutisches Mittel, zur Verwendung in der Behandlung von Krebserkrankungen bei einem Zielorganismus.

10. Therapeutischer Nanopartikel und chemotherapeutisches Mittel zur Verwendung nach Anspruch 9, wobei die Krebserkrankung aus der Gruppe ausgewählt ist, die aus den folgenden besteht: Brustkrebs, Darmkrebs, einem Nierenkarzinom, Lungenkrebs, Hautkrebs, Eierstockkrebs, Bauchspeicheldrüsenkrebs, einem Prostatakarzinom, einem rektalen Karzinom, Magenkrebs, Schilddrüsenkrebs und einem Uteruskarzinom.

11. Therapeutischer Nanopartikel und chemotherapeutisches Mittel zur Verwendung nach Anspruch 9, wobei diese weiterhin eine Bildgebung eines Gewebes des Zielorganismus umfasst, um einen Aufenthaltsort oder eine Anzahl an Krebszellen bei dem Zielorganismus oder einen Aufenthaltsort der therapeutischen Nanopartikel bei dem Zielorganismus zu bestimmen.

12. Therapeutischer Nanopartikel und chemotherapeutisches Mittel zur Verwendung nach Anspruch 9, wobei der therapeutische Nanopartikel dem Zielorganismus in zwei oder mehr Dosisgaben verabreicht wird, wobei der therapeutische Nanopartikel dem Zielorganismus mindestens einmal wöchentlich verabreicht wird.

13. Therapeutischer Nanopartikel und chemotherapeutisches Mittel zur Verwendung nach Anspruch 9, wobei der therapeutische Nanopartikel dem Zieloerganismus durch intravenöse, subkutane, intraarterielle, intramuskuläre oder intraperitoneale Verabreichung verabreicht wird.

14. Therapeutischer Nanopartikel und chemotherapeutisches Mittel zur Verwendung nach Anspruch 9, wobei der Zielorganismus Bauchspeicheldrüsenkrebs hat.

15. Therapeutischer Nanopartikel und chemotherapeutisches Mittel zur Verwendung nach Anspruch 9, wobei es sich bei dem chemotherapeutischen Mittel um Gemcitabin handelt.

## Revendications

1. Nanoparticule thérapeutique, **caractérisée en ce que** ladite nanoparticule :
a un diamètre compris entre 10 nm et 30 nm ;
comprend un noyau d'oxyde de fer, un enrobage polymère comprenant du dextrane et un acide nucléique inhibiteur ciblant une molécule de point de contrôle immunitaire, la molécule de point de contrôle immunitaire étant le ligand 1 de mort cellulaire programmée 1 (PD-L1), qui est lié de manière covalente à la nanoparticule.

2. Nanoparticule thérapeutique selon la revendication 1, dans laquelle l'acide nucléique comprend une séquence d'au moins 10 nucléotides contigus complémentaires de SEQ ID NO : 1.

3. Nanoparticule thérapeutique selon la revendication 1, dans laquelle l'acide nucléique comprend au moins un nucléotide modifié, le ou les nucléotides modifiés comprenant au moins une modification de leur base et/ou au moins une modification de leur sucre, ou le ou les nucléotides modifiés comprenant une modification d'un atome qui forme une liaison phosphodiester entre deux nucléotides adjacents dans l'acide nucléique.

4. Nanoparticule thérapeutique selon la revendication 3, dans laquelle le ou les nucléotides modifiés sont des nucléotides bloqués.

5. Nanoparticule thérapeutique selon la revendication 1, dans laquelle l'acide nucléique est une molécule de petit ARN interférent (siRNA).

6. Nanoparticule thérapeutique selon la revendication 1, dans laquelle l'acide nucléique est lié de manière covalente à la nanoparticule par l'intermédiaire d'un fragment chimique comprenant une liaison disulfure ou une liaison thioéther.

7. Nanoparticule thérapeutique selon la revendication 1, la nanoparticule étant magnétique.

8. Composition pharmaceutique comprenant la nanoparticule thérapeutique selon l'une quelconque des revendications 1 à 7, et éventuellement un agent chimiothérapeutique, l'agent étant éventuellement la gemcitabine.

9. Nanoparticule thérapeutique selon les revendications 1 à 7, et agent chimiothérapeutique, pour une utilisation dans le traitement du cancer chez un sujet.

10. Nanoparticule thérapeutique et agent chimiothérapeutique pour une utilisation selon la revendication 9, le cancer étant choisi dans le groupe constitué par : le cancer du sein, le cancer du côlon, le cancer du rein, le cancer du poumon, le cancer de la peau, le cancer de l'ovaire, le cancer du pancréas, le cancer de la prostate, le cancer rectal, le cancer de l'estomac, le cancer de la thyroïde et le cancer de l'utérus.

11. Nanoparticule thérapeutique et agent chimiothérapeutique pour une utilisation selon la revendication 9, comprenant en outre l'imagerie d'un tissu du sujet afin de déterminer la localisation ou le nombre de cellules cancéreuses chez le sujet, ou la localisation des nanoparticules thérapeutiques chez le sujet.

12. Nanoparticule thérapeutique et agent chimiothérapeutique pour une utilisation selon la revendication 9, la nanoparticule thérapeutique étant administrée en au moins deux doses au sujet, la nanoparticule thérapeutique étant éventuellement administrée au sujet au moins une fois par semaine.

13. Nanoparticule thérapeutique et agent chimiothérapeutique pour une utilisation selon la revendication 9, administrés au sujet par voie intraveineuse, sous-cutanée, intra-artérielle, intramusculaire ou intrapéritonéale.

14. Nanoparticule thérapeutique et agent chimiothérapeutique pour une utilisation selon la revendication 9, le sujet étant atteint d'un cancer du pancréas.

15. Nanoparticule thérapeutique et agent chimiothérapeutique pour une utilisation selon la revendication 9, l'agent chimiothérapeutique étant la gemcitabine.
